# EUROPEAN PATENT APPLICATION

(11) **EP 4 772 626 A1**
(43) Date of publication of application: **08.07.2026**
(21) Application number: 24859901.1
(22) Date of filing: 29.08.2024
(51) Int. Cl.: C12N 15/09, C12N 15/63

(54) **GENOME EDITING TECHNIQUE**

(30) Priority: 30.08.2023 JP 2023140063
(71) Applicant: The School Corporation Kansai University, Suita-shi, Osaka 564-8680 (JP); JNC Corporation, Chiyoda-ku Tokyo 100-8105 (JP)
(72) Inventor: YAMANAKA, Kazuya, Suita-shi, Osaka 564-8680 (JP); SAKURAI, Nobuki, Yokohama-shi, Kanagawa 236-8605 (JP)
(74) Representative: Becker, Eberhard
(86) International application number: PCT/JP2024/030955
(87) International publication number: WO 2025/047872

(57) **Abstract**

Provided is a method for deleting, substituting or inserting a target nucleotide sequence in a genome editing target region in an organism or a cell to be subjected to genome editing. The method comprises: (1) a step for introducing a type II restriction endonuclease recognition sequence into the genome editing target region; and (2) a step for expressing a type II restriction endonuclease capable of recognizing the introduced type II restriction endonuclease recognition sequence in the organism or the cell to cause double strand break (DSB) in the genome. In the method, after performing the steps (1) and (2), the target region is edited through homologous recombination repair with the genome editing gene sequence in the organism or the cell. In the organism or the cell, the GC content in the genome is 65% or more. As a result, it becomes possible to provide a genome editing technique for all organisms, particularly actinomycetes, wherein an organism or a cell obtained after the modification does not correspond to a recombinant and an off-target effect can be avoided.

## Description

### Technical Field

The present invention relates to a genome editing technique for organisms in general, particularly actinomycetes.

### Background Art

Actinomycetes have played an important role in the pharmaceutical industry field since ancient times because actinomycetes produce a wide variety of drug seed compounds as secondary metabolites. However, since secondary metabolites are not essential for the growth of the producing bacteria themselves, the production amounts thereof are extremely small. Furthermore, since multiple metabolites are also co-produced, intensive research has been conducted in breeding high-production mutant strains through genome modification, such as closure of co-producing secondary metabolic pathways and modification of metabolic fluxes, with the aim of improving the production of main products.

The homologous recombination method is widely known as a genome modification technique, for example. However, while the homologous recombination method can modify the target region with high specificity, foreign genes remain in the modified genome because the target gene and drug resistance gene are replaced. Therefore, the modified organism or cell corresponds to a recombinant. Consequently, it has been difficult to gain public acceptance for actual commercial production, and since there are also limitations on drug resistance genes available for genome modification, modification of multiple gene regions, so-called multiplex modification, is also difficult. It is widely known that multiplex modification can be performed by using the subsequently developed Cre-LoxP system, but LoxP sites remain in the modified genome, and the problem of corresponding to a recombinant remains unresolved.

On the other hand, the genome editing system using CRISPR-Cas9, which has attracted attention in recent years, allows any gene region to be deleted on a large scale without leaving heterologous organism-derived sequences on the modified genome. However, sequence recognition by Cas9 nuclease, which plays a role in introducing double-strand breaking (DSB) into the genome, requires a protospacer-adjacent motif (PAM), and the PAM of the commonly used Cas9 derived from Streptococcus pyogenes is G-rich and has 3 nucleotides (5'-NGG-3'). Nevertheless, this sequence is distributed throughout the actinomycete genome with a GC content of approximately 70%, and is present at 260 sites per 1000 bp in the *Streptomyces coelicolor* genome (Non-Patent Document 1). Therefore, non-specific genome cleavage leads to toxicity expression and frequently causes off-target effects, which makes the application difficult. To address this technical issue, an actinomycete-specific genome editing technique using Cas9 and Cpf1 derived from Francisella novicida, which recognizes T-rich PAM sequences (5'-TTV-3'), was developed (Non-Patent Document 1). However, the distribution of this PAM sequence in the actinomycete genome is limited, and it is difficult to specifically guide the nuclease to the editing target region, so this technique was not widely used (Non-Patent Document 2).

To achieve selective editing of desired regions in the actinomycete genome with high GC content and many repetitive sequences, a nuclease that can specifically introduce DSB into the editing target region without being affected by GC content or repetitive sequences is required. A nuclease that could serve as a potential candidate is the homing endonuclease, which is involved in RNA splicing in eukaryotes. Since homing endonucleases recognize extremely long nucleotide sequences (14 to 40 bp) (Non-Patent Document 3), introduction of specific DSB can be achieved by introducing a recognition sequence that does not exist in the genomic DNA into the editing target region in advance, and therefore it is actually applied as a genome editing technique (Non-Patent Document 4). On the other hand, in order to accurately cleave introns and inteins while avoiding toxicity even when target or host organism sequences diversify, homing endonucleases have the property of low stringency of recognition sequences (Non-Patent Document 3). Therefore, they may still cleave with differences of about several nucleotides, so it is considered difficult to completely avoid the problem of causing off-target effects, similar to Cas9. For this reason, there are no examples of a genome editing technique using the homing endonuclease being applied to genome editing of actinomycetes with high GC content.

### Related Art Documents

### Patent Documents

Patent Document 1: Japanese Patent Application Laid-Open No. 2005-237335

### Non-Patent Documents

Non-Patent Document 1: Appl Environ Microbiol. 2018 Sep 15;84(18):e00827-18
Non-Patent Document 2: Biomolecules. 2020 May 8;10(5):734
Non-Patent Document 3: Quarterly reviews of biophysics. 2005;38(1):49-95
Non-Patent Document 4: Applied microbiology and biotechnology. 2020;104(8):3597-609

### SUMMARY OF INVENTION

### Problem to be Solved by the Invention

The present invention provides a genome editing technique for organisms in general, particularly actinomycetes, wherein the modified organism or cell does not correspond to a recombinant and the off-target effects can be avoided.

### Means for Solving the Problem

As a result of intensive studies to solve the above problem, the inventors conceived of introducing a type II restriction endonuclease recognition sequence that does not exist in the genome into an editing target region of the genome, and introducing a type II restriction endonuclease that recognizes the type II restriction endonuclease recognition sequence from outside into the organism or cell. Thereby, the inventors found that double-strand breaking (DSB) can be induced in the DNA at the editing target region, and that by utilizing the function of DSB repair based on homologous recombination that the organism itself possesses, deletion, insertion, and substitution of DNA sequences can be achieved in the editing target region, leading to the present invention.

That is, the present invention is as follows.
[1] A method for deleting, substituting or inserting a target nucleotide sequence in a genome editing target region in an organism or cell to be subjected to genome editing, the method including:
   (1) a step of introducing a type II restriction endonuclease recognition sequence into the genome editing target region; and
   (2) a step of expressing a type II restriction endonuclease that recognizes the introduced type II restriction endonuclease recognition sequence in the organism or cell to induce double-strand breaking (DSB) in a genome,

   wherein after performing the steps (1) and (2), a target region is edited through homologous recombination repair with a genome editing gene sequence in the organism or cell, and
   a GC content in the genome of the organism or cell is 65% or more.
[2] The method according to [1], wherein the type II restriction endonuclease recognizes a nucleotide sequence composed only of A and T.
[3] The method according to [1] or [2], wherein the type II restriction endonuclease is PacI.
[4] The method according to any one of [1] to [3], wherein the organism is a eukaryote or a prokaryote that does not have the type II restriction endonuclease recognition sequence in the genome.
[5] The method according to any one of [1] to [4], wherein the organism is an actinomycete.
[6] The method according to [5], wherein the actinomycete is an actinomycete that does not have a recognition sequence for PacI in the genome.
[7] The method according to [5] or [6], wherein the actinomycete is an actinomycete selected from the group consisting of genus Streptomyces, genus Saccharopolyspora, and genus Amycolaptosis.
[8] The method according to any one of [5] to [7], wherein the actinomycete is an actinomycete selected from the group consisting of *Streptomyces fradiae*, *Streptomyces venezuelae, Streptomyces griseus, Streptomyces albulus, Streptomyces coelicolor, Streptomyces rimosus*, *Streptomyces spectabilis*, *Streptomyces avermitilis*, *Amycolatopsis orientalis*, and *Saccharopolyspora spinosa.*
[9] The method according to any one of [1] to [8], wherein introduction in the step (1) is performed using an introduction vector for the type II restriction endonuclease recognition sequence, and
   the introduction vector is a vector including (i) the genome editing gene sequence, (ii) the type II restriction endonuclease recognition sequence, (iii) an origin of replication sequence that operates in a host organism used for cloning the introduction vector and does not operate in the organism or cell to be subjected to genome editing, and (iv) a drug resistance gene sequence.
[10] The method according to [9], wherein the origin of replication sequence is a p15A ori sequence.
[11] The method according to [9] or [10], wherein the introduction vector further includes a constitutive expression promoter sequence in the organism or cell to be subjected to genome editing and a negative selection gene sequence operably arranged downstream thereof, and optionally, the negative selection gene sequence is codon-optimized.
[12] The method according to [11], wherein the constitutive expression promoter sequence is an ermE promoter (PermE) sequence, and/or
   the negative selection gene sequence is a codA gene sequence.
[13] The method according to [11] or [12], further including:
   (3) a step of selecting a cell into which the type II restriction endonuclease recognition sequence has been introduced using the negative selection gene.
[14] The method according to any one of [1] to [13], wherein the step (2) is performed using an expression vector for the type II restriction endonuclease that recognizes the introduced type II restriction endonuclease recognition sequence, and
   the expression vector is a vector including a promoter sequence that operates in the organism or cell to be subjected to genome editing and a type II restriction endonuclease sequence operably arranged downstream thereof, and
   including an origin of replication sequence that operates in a host organism used for cloning the expression vector and does not operate in the organism or cell to be subjected to genome editing, a drug resistance gene sequence, and a sequence of temperature sensitive origin of replication that operates in the organism or cell to be subjected to genome editing.
[15] The method according to [14], wherein the promoter sequence is an inducible expression promoter sequence,
   the origin of replication sequence that operates in the host organism used for cloning the expression vector and does not operate in the organism or cell to be subjected to genome editing is a pUC ori sequence, and/or
   the sequence of the temperature sensitive origin of replication that operates in the organism or cell to be subjected to genome editing is a pSG5rep sequence.
[16] The method according to [15], wherein the inducible expression promoter sequence is a tipA promoter (PtipA) sequence.
[17] The method according to any one of [14] to [16], wherein the expression vector includes an operator sequence and a repressor sequence,
   the operator sequence is inserted between the promoter sequence and a transcription start site, and
   optionally, the type II restriction endonuclease sequence is codon-optimized.
[18] The method according to [17], wherein the operator sequence is a lacO sequence, and/or
   the repressor sequence is a lacI sequence.
[19] The method according to any one of [14] to [18], further including:
   (4) a step of eliminating the expression vector from the organism or cell based on temperature sensitivity of the temperature sensitive origin of replication.
[20] The method according to any one of [1] to [8], being a method for performing introduction of the type II restriction endonuclease recognition sequence in the step (1) and expression of the type II restriction endonuclease that recognizes the type II restriction endonuclease recognition sequence in the step (2) with a single vector,
   wherein the single vector is a vector including (i) the genome editing gene sequence, (ii) the type II restriction endonuclease recognition sequence, (iii) a promoter sequence that operates in the organism or cell to be subjected to genome editing and a type II restriction endonuclease sequence operably arranged downstream thereof, (iv) an origin of replication sequence that operates in a host organism used for cloning the single vector and does not operate in the organism or cell to be subjected to genome editing, and (v) a drug resistance gene sequence.
[21] The method according to [20], wherein the promoter sequence is an inducible expression promoter sequence, and/or
   the origin of replication sequence that operates in the host organism used for cloning the single vector and does not operate in the organism or cell to be subjected to genome editing is a pUC ori sequence.
[22] The method according to [21], wherein the inducible expression promoter sequence is a tipA promoter (PtipA) sequence.
[23] The method according to any one of [20] to [22], wherein the single vector includes an operator sequence and a repressor sequence,
   the operator sequence is inserted between the promoter sequence and a transcription start site, and
   optionally, the type II restriction endonuclease sequence is codon-optimized.
[24] The method according to [23], wherein the operator sequence is a lacO sequence, and/or
   the repressor sequence is a lacI sequence.
[25] An introduction vector for type II restriction endonuclease recognition sequence, including (i) a genome editing gene sequence or a region into which the genome editing gene sequence is insertable, (ii) a type II restriction endonuclease recognition sequence, (iii) an origin of replication sequence that operates in a host organism used for cloning the introduction vector and does not operate in an organism or cell to be subjected to genome editing, and (iv) a drug resistance gene sequence.
[26] The introduction vector according to [25], wherein the origin of replication sequence is a p15A ori sequence.
[27] The introduction vector according to [25] or [26], further including a constitutive expression promoter sequence in the organism or cell to be subjected to genome editing and a negative selection gene sequence operably arranged downstream thereof, and
   optionally, the negative selection gene sequence is codon-optimized.
[28] The introduction vector according to [27], wherein the constitutive expression promoter sequence is an ermE promoter (PermE) sequence, and/or
   the negative selection gene sequence is a codA gene sequence.
[29] An expression vector for type II restriction endonuclease, including a promoter sequence that operates in an organism or cell to be subjected to genome editing and a type II restriction endonuclease sequence operably arranged downstream thereof, and
   including an origin of replication sequence that operates in a host organism used for cloning the expression vector and does not operate in the organism or cell to be subjected to genome editing, a drug resistance gene sequence, and a temperature sensitive origin of replication sequence that operates in the organism or cell to be subjected to genome editing.
[30] The expression vector according to [29], wherein the promoter sequence is a tipA promoter (PtipA) sequence,
   the origin of replication sequence that operates in the host organism used for cloning the expression vector and does not operate in the organism or cell to be subjected to genome editing is a pUC ori sequence, and/or
   the temperature sensitive origin of replication sequence that operates in the organism or cell to be subjected to genome editing is a pSG5rep sequence.
[31] The expression vector according to [29] or [30], the expression vector includes an operator sequence and a repressor sequence,
   the operator sequence is inserted between the promoter sequence and a transcription start site, and
   optionally, the type II restriction endonuclease sequence is codon-optimized.
[32] The expression vector according to [31], wherein the operator sequence is a lacO sequence, and/or
   the repressor sequence is a lacI sequence.
[33] A single vector for introducing a type II restriction endonuclease recognition sequence and for expressing a type II restriction endonuclease, including (i) a genome editing gene sequence or a region into which the genome editing gene sequence is insertable, (ii) a type II restriction endonuclease recognition sequence, (iii) a promoter sequence that operates in an organism or cell to be subjected to genome editing and a type II restriction endonuclease sequence operably arranged downstream thereof, (iv) an origin of replication sequence that operates in a host organism used for vector cloning and does not operate in the organism or cell to be subjected to genome editing, and (v) a drug resistance gene sequence.
[34] The single vector according to [33], wherein the promoter sequence is a tipA promoter (PtipA) sequence, and/or
   the origin of replication sequence that operates in the host organism used for cloning the single vector and does not operate in the organism or cell to be subjected to genome editing is a pUC ori sequence.
[35] The single vector according to [33] or [34], wherein the single vector includes an operator sequence and a repressor sequence,
   the operator sequence is inserted between the promoter sequence and a transcription start site, and
   optionally, the type II restriction endonuclease sequence is codon-optimized.
[36] The single vector according to [35], wherein the operator sequence is a lacO sequence, and/or
   the repressor sequence is a lacI sequence.
[37] A kit for deleting, substituting or inserting a target nucleotide sequence in a genome editing target region in an organism or cell to be subjected to genome editing, the kit including:
   (i) the introduction vector according to any one of [25] to [28], and the expression vector according to any one of [29] to [32], or
   (ii) the single vector according to any one of [33] to [36].
[38] Use of the vector according to any one of [25] to [28] for introducing a type II restriction endonuclease recognition sequence into a genome editing target region in an organism or cell to be subjected to genome editing.
[39] Use of the vector according to any one of [29] to [32] for expressing a type II restriction endonuclease in an organism or cell to be subjected to genome editing.
[40] Use of the vector according to any one of [33] to [36] for introducing a type II restriction endonuclease recognition sequence into a genome editing target region in an organism or cell to be subjected to genome editing, and for expressing a type II restriction endonuclease in the organism or cell to be subjected to genome editing.
[41] Use of the kit according to [37] for deleting, substituting or inserting a target nucleotide sequence in a genome editing target region in an organism or cell to be subjected to genome editing.

### Effects of the Invention

According to the present invention, it is possible to provide a genome editing technique for organisms in general, particularly actinomycetes, wherein the modified organism or cell does not correspond to a recombinant and the off-target effects can be avoided. That is, since no foreign DNA remains in the edited genome, the organism or cell after genome editing does not correspond to a recombinant, and by using a type II restriction endonuclease that recognizes sequences not present in the genome of the microorganism to be edited with extremely high specificity, the off-target effects can be avoided.

### BRIEF DESCRIPTION OF THE DRAWINGS

[FIG. 1] shows a schematic diagram of genome editing when using two types of vectors.
[FIG. 2] shows a schematic diagram of the actinorhodin biosynthesis gene cluster.
[FIG. 3] shows a vector map of pPacIᵢₙₜ.
[FIG. 4] shows a vector map of pSET152-PtipA-gfpᵤᵥ-tsr.
[FIG. 5] shows a vector map of pSET152-PtipA-gfpᵤᵥ-pSG5rep.
[FIG. 6] is a diagram showing thiostrepton concentration-dependent GFP expression in Streptomyces lividans. The left panel is a photograph of cell-free extract of S. lividans expressing GFP in a thiostrepton concentration-dependent manner observed under ultraviolet irradiation, and the right panel is a graph showing fluorescence intensity.
[FIG. 7] is a diagram showing thiostrepton concentration-dependent GFP expression in *Streptomyces albulus.* The left panel is a photograph of cell-free extract of S. albulus expressing GFP in a thiostrepton concentration-dependent manner observed under ultraviolet irradiation, and the right panel is a graph showing fluorescence intensity.
[FIG. 8] is a diagram showing thiostrepton concentration-dependent GFP expression in *Streptomyces coelicolor.* The left panel is a photograph of cell-free extract of S. coelicolor expressing GFP in a thiostrepton concentration-dependent manner observed under ultraviolet irradiation, and the right panel is a graph showing fluorescence intensity.
[FIG. 9] shows a vector map of pSET152-PtipA(lacO)-gfp-tsr-pSG5rep.
[FIG. 10] shows a vector map of pSET152-PtipA(lacO)-gfp-tsr-pSG5rep-lacI.
[FIG. 11] shows a vector map of pPacIᵢₙₜΔact.
[FIG. 12] shows a construction scheme for the editing intermediate strain with a PacI recognition sequence inserted into the genome.
[FIG. 13] is a photograph showing transformants obtained by introducing pELIM into editing intermediate strain.
[FIG. 14] is a photograph confirming actinorhodin production by liquid culture.
[FIG. 15] the upper panel shows a schematic diagram of the editing intermediate, editing completion strain, and wild-type revertant strain. The lower panel shows photographs of the phenotype of transformant (left) and genotype analysis (right).
[FIG. 16] is a sequence analysis diagram of the region surrounding the genome editing region in the actinorhodin biosynthesis gene cluster.
[FIG. 17] shows a vector map of pPacIᵢₙₜΔactVB.
[FIG. 18] is a diagram showing genotype analysis of the pELIM-introduced strain. The upper panel shows a schematic diagram of actVB deletion. The lower panel shows a photograph of genotype analysis in each sample.
[FIG. 19] is a sequence analysis diagram of the region surrounding the genome editing region of the editing region in actVB deletion.
[FIG. 20] is a schematic diagram of genome editing when using a single vector (right), and a photograph showing the results of restriction enzyme treatment of the constructed vector (pELIM-Δact1) (left).
[FIG. 21] is a schematic diagram of the editing intermediate, editing completion strain, and wild-type revertant strain (left), and a photograph confirming actinorhodin production by liquid culture (right). In the photograph, the right tube shows the desired editing completion strain, and the left shows the wild-type revertant strain.

### DESCRIPTION OF THE EMBODIMENTS

### <Genome Editing Method>

One embodiment of the present invention is,
a method for deleting, substituting or inserting a target nucleotide sequence in a genome editing target region in an organism or cell to be subjected to genome editing, the method including:
   (1) a step of introducing a type II restriction endonuclease recognition sequence into the genome editing target region; and
   (2) a step of expressing a type II restriction endonuclease that recognizes the introduced type II restriction endonuclease recognition sequence in the organism or cell to induce double-strand breaking (DSB) in a genome,
wherein after performing steps (1) and (2), a target region is edited through homologous recombination repair with a genome editing gene sequence in the organism or cell, and
a GC content in the genome of the organism or cell is 65% or more.

In the present invention, genome editing refers to deletion, substitution or insertion of a target nucleotide sequence in a genome editing target region. The genome editing target region refers to a region including a target gene to be genome edited, and may be a region consisting only of the target gene or may be a region further including a region in the vicinity of the target gene.

In the present invention, the target organism or cell is not particularly limited as long as the GC content in the genome of the organism or cell is high. In the present invention, high GC content means that the proportion of G and C in the genome is 65% or more, and may be 68% or more, may be 70% or more or may be 72% or more.

The target organism or cell is, for example, a eukaryote or prokaryote that does not have the type II restriction endonuclease recognition sequence in the genome.

Examples of organisms with high GC content in the genome include actinomycetes, which can be suitably used in the genome editing method of the present invention.

The actinomycetes are not particularly limited as long as the actinomycetes can be subjected to genome editing according to the present invention, but preferably are actinomycetes that do not have a recognition sequence for PacI in the genome.

As the actinomycetes, for example, actinomycetes selected from the group consisting of the genus Streptomyces, the genus Saccharopolyspora, and the genus Amycolaptosis can be used.

Examples of bacteria belonging to the genus Streptomyces include *Streptomyces fradiae*, *Streptomyces venezuelae, Streptomyces griseus, Streptomyces albulus, Streptomyces coelicolor, Streptomyces rimosus*, *Streptomyces spectabilis*, *Streptomyces avermitilis*, and the like.

Examples of bacteria belonging to the genus Saccharopolyspora include *Saccharopolyspora spinosa* and the like.

Examples of bacteria belonging to the genus Amycolatopsis include *Amycolatopsis orientalis* and the like.

For example, in the case of using PacI described below as a type II restriction endonuclease, the target organism or cell preferably does not have a PacI recognition sequence (that is, PacI site) in the genome thereof. Examples of such bacteria include *Streptomyces fradiae*, *Streptomyces venezuelae, Streptomyces griseus, Streptomyces albulus, Streptomyces coelicolor, Streptomyces rimosus, Streptomyces spectabilis, Streptomyces avermitilis*, *Saccharopolyspora spinosa*, *Amycolatopsis orientalis*, and the like.

In the present invention, the type II restriction endonuclease is not particularly limited as long as the type II restriction endonuclease strictly recognizes a desired sequence. Here, the type II restriction endonuclease recognizes sequences extremely strictly and can cleave double-stranded DNA. Therefore, by using the type II restriction endonuclease, off-target effects can be avoided. Additionally, since non-specific genome cleavage does not occur, toxicity expression can also be avoided.

For example, in the case of actinomycetes being the target organism for genome editing, since the GC content in the genome is high, a type II restriction endonuclease that recognizes a nucleotide sequence composed only of A and T can be suitably used.

For example, PacI derived from Pseudomonas alcaligenes can be used as a type II restriction endonuclease. The sequence recognized by PacI is a palindromic double-stranded nucleotide sequence composed only of A and T (that is, 5'-TTAATTAA-3') that does not exist in many actinomycete genomes. Since PacI is the smallest among type II restriction endonucleases (number of amino acid residues: 142aa), PacI has the advantage that the expression vector can be miniaturized and transformation can be easily performed.

Step (1) in the present embodiment is a step of introducing a type II restriction endonuclease recognition sequence into the genome editing target region.

The method of the introduction is not particularly limited, but can be performed using, for example, an introduction vector for type II restriction endonuclease recognition sequence. By introducing the introduction vector for type II restriction endonuclease recognition sequence into a host, an editing intermediate can be obtained in which the full length of the vector including the type II restriction endonuclease recognition sequence is inserted into the genome by single crossover homologous recombination.

Introduction into the host can be performed by methods known to those skilled in the art, and can be performed, for example, by conjugative transfer via escherichia coli (E. coli ET12567).

As the introduction vector for type II restriction endonuclease recognition sequence, a vector can be used that includes (i) a genome editing gene sequence, (ii) a type II restriction endonuclease recognition sequence, (iii) an origin of replication sequence that operates in a host organism used for cloning the introduction vector and does not operate in the organism or cell to be subjected to genome editing, and (iv) a drug resistance gene sequence.

The genome editing gene sequence refers to the desired edited sequence in the genome editing target region, that is, the sequence after deletion, substitution or insertion of the target nucleotide sequence in the genome editing target region. For example, in the case of deleting a target nucleotide sequence by genome editing, the genome editing gene sequence refers to a sequence in which the flanking regions on the 5' side and 3' side of the target nucleotide sequence in the genome editing target region are linked. It is noted that the linked sequence does not preclude including regions other than the protein coding region in the gene in the case of performing genome editing for the purpose of functional loss (inactivation) of a certain gene.

The length of the sequence of the flanking region is arbitrary, but for example, the lengths of the regions on the 5' side and 3' side may each be approximately 1 kbp, may be 100 bp to 2 kbp, may be 500 bp to 1.5 kbp, and may be 800 bp to 1.2 kbp.

Further, for example, in the case of substituting a target nucleotide sequence by genome editing, a sequence can be used in which the substituted sequence is included and linked between the flanking regions on the 5' side and 3' side of the target nucleotide sequence in the genome editing target region.

Further, for example, in the case of inserting a target nucleotide sequence by genome editing, a sequence can be used in which the sequence to be inserted is included and linked between the flanking regions on the 5' side and 3' side of the target nucleotide sequence in the genome editing target region.

As described above, a nucleotide sequence composed only of A and T can be suitably used as the type II restriction endonuclease recognition sequence, and furthermore, a PacI recognition sequence (that is, 5'-TTAATTAA-3') can be more suitably used.

In addition, the introduction vector includes a p15A ori sequence, which is an origin of replication sequence in escherichia coli, and a drug resistance gene sequence.

Due to the inclusion of an origin of replication sequence that operates in the host organism used for cloning the introduction vector and does not operate in the organism or cell to be subjected to genome editing, the introduction vector does not autonomously replicate within the organism or cell to be subjected to genome editing. In the case of using escherichia coli for replicating the introduction vector, it is preferable to use an origin of replication sequence suitable for replication in escherichia coli, and for example, a p15A ori sequence can be used. The p15A ori sequence may have, for example, the nucleotide sequence described in SEQ ID NO: 68, and may have a nucleotide sequence that is 90% or more identical, 95% or more identical or 98% or more identical to the nucleotide sequence described in SEQ ID NO: 68, as long as the function as p15A ori is not lost.

The drug resistance gene sequence is not particularly limited, but may be, for example, a drug resistance gene against kanamycin, apramycin, neomycin, and the like. Multiple drug resistance gene sequences may be included as required.

The introduction vector may include a negative selection gene sequence. By introducing a negative selection gene into the editing intermediate strain, unedited intermediate strains can be eliminated and editing completion strains can be selectively obtained. For example, escherichia coli-derived cytosine deaminase (codA) gene is known as a negative selection gene that can be used in actinomycetes. The negative selection gene is not particularly limited, but for example, codA gene, SacB gene or the like can be used. The codA gene may have, for example, a nucleotide sequence encoding codA having the amino acid sequence described in SEQ ID NO: 69. Further, as long as codA does not lose the function as a negative selection marker, codA may have an amino acid sequence that is 90% or more identical, 95% or more identical or 98% or more identical to the amino acid sequence described in SEQ ID NO: 69.

Furthermore, the negative selection gene sequence may be codon-optimized, and translation efficiency within actinomycetes can be enhanced by codon optimization. For example, when using codA gene as a negative selection gene, the codon-optimized nucleotide sequence described in SEQ ID NO: 70 can be listed (codAsm).

It is preferable that the negative selection gene has a constitutive expression promoter sequence in the organism or cell to be subjected to genome editing, which is operably arranged upstream thereof.

An ermE promoter (PermE) sequence can be used as the constitutive expression promoter sequence. The PermE sequence may have, for example, the nucleotide sequence described in SEQ ID NO: 71, and may have a nucleotide sequence that is 90% or more identical, 95% or more identical or 98% or more identical to the nucleotide sequence described in SEQ ID NO: 71, as long as the function as a promoter is not lost.

The principle of negative selection by codA is as follows.

The codA gene is a gene that functions as a negative selection marker in the presence of 5-fluorocytosine (5-FC) in actinomycetes.

5-Fluorocytosine is harmless, but is converted to toxic 5-fluorouracil by codA. codA is constantly expressed by the ermE promoter in actinomycetes. In the case of adding 5-fluorocytosine to the medium, strains having codA cannot grow, and only strains not containing codA grow. Negative selection by codA can be used for elimination of editing intermediate strains that remain at a certain frequency during the genome editing process.

The negative selection can be performed at any stage after step (2), but on the other hand, negative selection may not be performed. By performing negative selection, the abundance ratio of editing completion strains can be increased, that is, editing intermediate strains can be reduced.

Accordingly, the genome editing method of the present embodiment may further include (3) a step of selecting a cell into which the type II restriction endonuclease recognition sequence has been introduced using the negative selection gene.

The introduction vector may include other genes as required.

Only one type of introduction vector may be used, but multiple types may be used as required, and it is possible to perform multiplex genome modification by using multiple types.

Step (2) in the present embodiment is a step of expressing a type II restriction endonuclease that recognizes the introduced type II restriction endonuclease recognition sequence in the organism or cell to induce double-strand breaking (DSB) in the genome.

The method of the expression is not particularly limited, but for example, can be performed using an expression vector for type II restriction endonuclease that recognizes the introduced type II restriction endonuclease recognition sequence.

Introduction into the host can be performed by methods known to those skilled in the art, and can be performed, for example, by conjugative transfer via escherichia coli (E. coli ET12567).

As the expression vector for type II restriction endonuclease, a vector can be used that includes a promoter sequence that operates in the organism or cell to be subjected to genome editing and a type II restriction endonuclease sequence operably arranged downstream thereof, and includes an origin of replication sequence that operates in a host organism used for cloning the expression vector and does not operate in the organism or cell to be subjected to genome editing, a drug resistance gene sequence, and a sequence of temperature sensitive origin of replication that operates in the organism or cell to be subjected to genome editing.

As the promoter sequence that operates in the organism or cell to be subjected to genome editing, for example, an inducible expression promoter sequence can be used, and particularly a thiostrepton-inducible promoter (PtipA) sequence can be suitably used. The thiostrepton-inducible promoter is a promoter that induces expression of a gene operably arranged downstream thereof in the presence of thiostrepton. The thiostrepton-inducible promoter may have, for example, the nucleotide sequence described in SEQ ID NO: 72, and may have a nucleotide sequence that is 90% or more identical, 95% or more identical or 98% or more identical to the nucleotide sequence described in SEQ ID NO: 72, as long as the function as a thiostrepton-inducible promoter is not lost.

The type II restriction endonuclease sequence is a sequence of a type II restriction endonuclease that recognizes the type II restriction endonuclease recognition sequence introduced in the introduction vector. For example, when introducing a PacI recognition sequence as a type II restriction endonuclease recognition sequence in the introduction vector, a PacI sequence is expressed in the host as the type II restriction endonuclease sequence in the expression vector. The PacI sequence may have, for example, the nucleotide sequence described in SEQ ID NO: 22, and may have a nucleotide sequence that is 90% or more identical, 95% or more identical or 98% or more identical to the nucleotide sequence described in SEQ ID NO: 22, as long as the function as PacI is not lost.

Furthermore, the type II restriction endonuclease sequence may be codon-optimized, and translation efficiency within actinomycetes can be enhanced by codon optimization.

Due to the inclusion of an origin of replication sequence that operates in the host organism used for cloning the expression vector and does not operate in the organism or cell to be subjected to genome editing, it does not function within the organism or cell to be subjected to genome editing. In the case of using escherichia coli for replicating the expression vector, it is preferable to use an origin of replication sequence suitable for replication in escherichia coli, and for example, a pUC ori sequence can be used. The pUC ori sequence may have, for example, the nucleotide sequence described in SEQ ID NO: 73, and may have a nucleotide sequence that is 90% or more identical, 95% or more identical or 98% or more identical to the nucleotide sequence described in SEQ ID NO: 73, as long as the function as a pUC ori sequence is not lost.

The drug resistance gene is not particularly limited and can include any sequence.

For example, in the case of using a thiostrepton-inducible promoter as the promoter in the expression vector, it is preferable to include a thiostrepton resistance gene (tsr) as the drug resistance gene sequence. Multiple drug resistance gene sequences may be included as required, and for example, drug resistance genes against kanamycin, apramycin, neomycin, and the like may be included.

For example, a pSG5rep gene sequence, which is the origin of replication of the temperature sensitive plasmid pSG5, can be used as the sequence of temperature sensitive origin of replication that operates in the organism or cell to be subjected to genome editing. A vector including the pSG5rep gene sequence loses function at 39°C or higher, so plasmid replication does not occur. Therefore, during cell division, the plasmid is not transmitted to daughter cells, and as culture progresses, almost all cells become cells that do not contain the plasmid. By utilizing this property, after genome editing, vectors introduced from outside that have become unnecessary can be efficiently eliminated from the host.

A repressor sequence overexpressed in escherichia coli can control gene expression directly downstream of the promoter by binding to an operator sequence inserted between the promoter sequence and the transcription start site. Therefore, in the expression vector, it is preferable to include a repressor sequence and an operator sequence in order to suppress expression of the type II restriction endonuclease gene at the basal level. lacI can be used as the repressor sequence, and a lacO sequence can be used as the operator sequence.

Accordingly, the genome editing method of the present embodiment may further include (4) a step of eliminating the expression vector from the organism or cell based on the temperature sensitivity of the temperature sensitive origin of replication.

The expression vector may include other genes as required.

Only one type of expression vector may be used, but multiple types may be used as required.

The method for performing introduction of the type II restriction endonuclease recognition sequence in step (1) and expression of the type II restriction endonuclease that recognizes the type II restriction endonuclease recognition sequence in step (2) in the present embodiment is not particularly limited, but may be performed using a single vector. The single vector can be, for example, a vector including (i) a genome editing gene sequence, (ii) a type II restriction endonuclease recognition sequence, (iii) a promoter sequence that operates in the organism or cell to be subjected to genome editing and a type II restriction endonuclease sequence operably arranged downstream thereof, (iv) an origin of replication sequence that operates in a host organism used for cloning the single vector and does not operate in the organism or cell to be subjected to genome editing, and (v) a drug resistance gene sequence. In detail, the description regarding the above "introduction vector for type II restriction endonuclease recognition sequence" and the description regarding the "expression vector for type II restriction endonuclease that recognizes the introduced type II restriction endonuclease recognition sequence" can be incorporated by reference.

As the promoter sequence that operates in the organism or cell to be subjected to genome editing, for example, an inducible expression promoter sequence can be used, and particularly a thiostrepton-inducible promoter (PtipA) sequence can be suitably used. The thiostrepton-inducible promoter may have, for example, the nucleotide sequence described in SEQ ID NO: 72, and may have a nucleotide sequence that is 90% or more identical, 95% or more identical or 98% or more identical to the nucleotide sequence described in SEQ ID NO: 72, as long as the function as a thiostrepton-inducible promoter is not lost.

As the origin of replication sequence that operates in the host organism used for cloning the single vector and does not operate in the organism or cell to be subjected to genome editing, in the case of using escherichia coli for replicating the vector, it is preferable to use an origin of replication sequence suitable for replication in escherichia coli, and for example, a pUC ori sequence can be used. The pUC ori sequence may have, for example, the nucleotide sequence described in SEQ ID NO: 73, and may have a nucleotide sequence that is 90% or more identical, 95% or more identical or 98% or more identical to the nucleotide sequence described in SEQ ID NO: 73, as long as the function as a pUC ori sequence is not lost.

To suppress expression of the type II restriction endonuclease gene at the basal level, it is preferable to include a repressor sequence and an operator sequence. lacI can be used as the repressor sequence, and a lacO sequence can be used as the operator sequence. The operator sequence may be inserted between the promoter sequence and the transcription start site.

The type II restriction endonuclease sequence is not particularly limited as long as the above type II restriction endonuclease recognition sequence can be recognized, and may be codon-optimized.

The single vector may include other genes as required.

Only one type of single vector may be used, but multiple types may be used as required.

The genome editing method of the present embodiment may include additional steps as required.

### <Introduction Vector for Type II Restriction Endonuclease Recognition Sequence>

Another embodiment of the present invention is an introduction vector for type II restriction endonuclease recognition sequence, including (i) a genome editing gene sequence or a region into which the genome editing gene sequence is insertable, (ii) a type II restriction endonuclease recognition sequence, (iii) an origin of replication sequence that operates in a host organism used for cloning the introduction vector and does not operate in an organism or cell to be subjected to genome editing, and (iv) a drug resistance gene sequence.

The introduction vector of the present embodiment may have a genome editing gene sequence inserted, or any genome editing gene sequence may be inserted at the time of use as desired. Examples of the region into which the genome editing gene sequence is insertable include a region having a restriction enzyme sequence that can be treated with a desired restriction enzyme. It is noted that the restriction enzyme sequence preferably does not include the sequence contained in the vector.

In the introduction vector, the origin of replication sequence may be a p15A ori sequence.

The introduction vector further includes a constitutive expression promoter sequence in the organism or cell to be subjected to genome editing and a negative selection gene sequence operably arranged downstream thereof, and optionally, the negative selection gene sequence may be codon-optimized.

In the introduction vector, the constitutive expression promoter sequence may be an ermE promoter (PermE) sequence, and/or the negative selection gene sequence may be a codA gene sequence.

The introduction vector may optionally include other arbitrary nucleotide sequences as required.

Methods known to those skilled in the art can be used as the vector synthesis method. The synthesis method is not particularly limited as long as the desired vector can be synthesized. For example, a method may include amplifying a desired nucleotide sequence from a template sequence having the desired nucleotide sequence by PCR method such that an arbitrary restriction enzyme sequence is linked to the end, treating a plasmid with the arbitrary restriction enzyme, and synthesizing by linking the amplification product and the restriction enzyme-treated plasmid by Gibson assembly method.

In the present embodiment, the descriptions regarding the genome editing target and various sequences can incorporate by reference the description in the above <Genome Editing Method> section.

As another aspect of the present embodiment, use of the above vector for introducing a type II restriction endonuclease recognition sequence into a genome editing target region in an organism or cell to be subjected to genome editing can be listed.

### <Expression Vector for Type II Restriction Endonuclease>

Another embodiment of the present invention is,
an expression vector for type II restriction endonuclease, including a promoter sequence that operates in an organism or cell to be subjected to genome editing and a type II restriction endonuclease sequence operably arranged downstream thereof, and
including an origin of replication sequence that operates in a host organism used for cloning the expression vector and does not operate in the organism or cell to be subjected to genome editing, a drug resistance gene sequence, and a sequence of temperature sensitive origin of replication that operates in the organism or cell to be subjected to genome editing.

In the expression vector, the promoter sequence may be a tipA promoter (PtipA) sequence, the origin of replication sequence that operates in a host organism used for cloning the expression vector and does not operate in the organism or cell to be subjected to genome editing may be a pUC ori sequence, and/or the sequence of temperature sensitive origin of replication that operates in the organism or cell to be subjected to genome editing may be a pSG5rep sequence.

The expression vector includes an operator sequence and a repressor sequence, the operator sequence is inserted between the promoter sequence and the transcription start site, and optionally, the type II restriction endonuclease sequence may be codon-optimized.

In the expression vector, the operator sequence may be a lacO sequence, and/or the repressor sequence may be a lacI sequence.

The expression vector may optionally include other arbitrary nucleotide sequences as required.

Methods known to those skilled in the art can be used as the vector synthesis method. The synthesis method is not particularly limited as long as the desired vector can be synthesized. For example, a method may include amplifying a desired nucleotide sequence from a template sequence having the desired nucleotide sequence by PCR method such that an arbitrary restriction enzyme sequence is linked to the end, treating a plasmid with the arbitrary restriction enzyme, and synthesizing by linking the amplification product and the restriction enzyme-treated plasmid by Gibson assembly method.

In the present embodiment, the descriptions regarding the genome editing target and various sequences can incorporate by reference the descriptions in the above <Genome Editing Method> section.

As another aspect of the present embodiment, use of the above vector for expressing a type II restriction endonuclease in an organism or cell to be subjected to genome editing can be listed.

### <Single Vector>

Another embodiment of the present invention is a single vector for introducing a type II restriction endonuclease recognition sequence and for expressing a type II restriction endonuclease, including (i) a genome editing gene sequence or a region into which the genome editing gene sequence is insertable, (ii) a type II restriction endonuclease recognition sequence, (iii) a promoter sequence that operates in an organism or cell to be subjected to genome editing and a type II restriction endonuclease sequence operably arranged downstream thereof, (iv) an origin of replication sequence that operates in a host organism used for vector cloning and does not operate in the organism or cell to be subjected to genome editing, and (v) a drug resistance gene sequence.

In the single vector, the promoter sequence may be a tipA promoter (PtipA) sequence, and/or the origin of replication sequence that operates in the host organism used for cloning the single vector and does not operate in the organism or cell to be subjected to genome editing may be a pUC ori sequence.

The single vector includes an operator sequence and a repressor sequence, the operator sequence is inserted between the promoter sequence and the transcription start site, and optionally, the type II restriction endonuclease sequence may be codon-optimized.

In the single vector, the operator sequence may be a lacO sequence, and/or the repressor sequence may be a lacI sequence.

Methods known to those skilled in the art can be used as the vector synthesis method. The synthesis method is not particularly limited as long as the desired vector can be synthesized. For example, a method may include amplifying a desired nucleotide sequence from a template sequence having the desired nucleotide sequence by PCR method such that an arbitrary restriction enzyme sequence is linked to the end, treating a plasmid with the arbitrary restriction enzyme, and synthesizing by linking the amplification product and the restriction enzyme-treated plasmid by Gibson assembly method.

As another aspect of the present embodiment, use of the above vector for introducing a type II restriction endonuclease recognition sequence into a genome editing target region in an organism or cell to be subjected to genome editing, and for expressing a type II restriction endonuclease in the organism or cell to be subjected to genome editing can be listed.

### <Kit>

Another embodiment of the present invention is,
a kit for deleting, substituting or inserting a target nucleotide sequence in a genome editing target region in an organism or cell to be subjected to genome editing, the kit including (i) the vector described in the above <Introduction Vector for Type II Restriction Endonuclease Recognition Sequence> section and the vector described in the above <Expression Vector for Type II Restriction Endonuclease> section, or
(ii) the vector described in the above <Single Vector> section.

The kit of the present embodiment may additionally include arbitrary reagents and/or instruments. Examples of reagents may include primers, PCR reagents, controls, restriction enzymes, restriction enzyme treatment reagents, cloning host cells, media, diluents, buffers, water, purification reagents, and the like. Examples of instruments may include 96-well plates, reaction tubes, and the like.

In the present embodiment, the descriptions regarding the genome editing target and various vectors can incorporate by reference the descriptions in the above <Genome Editing Method>, <Introduction Vector for Type II Restriction Endonuclease Recognition Sequence>, <Expression Vector for Type II Restriction Endonuclease>, and <Single Vector> sections.

As another aspect of the present embodiment, use of the above kit for deleting, substituting or inserting a target nucleotide sequence in a genome editing target region in an organism or cell to be subjected to genome editing can be listed.

### Examples

Hereinafter, the present invention will be described more specifically based on examples, but the present invention is not limited to the following examples.

### <Example 1. Confirmation of the Number of PacI Sites in Actinomycete Genome and Examination of Genome Editing Principle>

Actinomycete genome is known to have high GC content. Therefore, theoretically, the frequency of occurrence of nucleotide sequences composed only of A and T with a certain length or more is extremely low.

For example, PacI derived from Pseudomonas alcaligenes is a type II restriction endonuclease that recognizes 5'-TTAATTAA-3'. Thus, the number of PacI sites in the genomes of major actinomycetes was examined. As a result, it was shown that the genomes of major actinomycetes did not have PacI sites or had very few even if they did, and PacI did not cleave the genomes of many types of actinomycetes (Table 1).

**[Table 1]**

| Table 1 Number of PacI sites in the genomes of model actinomycetes and major pharmaceutical lead compound production actinomycetes | | | | | | |
|---|---|---|---|---|---|---|
| Genus | Strain | Major secondary metabolites | Genome accession No. | GC content (%) | Genome size (Mbp) | Number of PacI sites |
| *Streptomyces* | *Streptomyces fradiae* ATCC 10745 | Neomycin | NZ-CP023696.1 | 75 | 6.7 | 0 |
| | *Streptomyces venezuelae* NBRL B-65442 | Chloramphenicol | NZ_CP018074.1 | 73 | 8.2 | 0 |
| | *Streptomyces griseus* NBRC13350 | Streptomycin | NC_010572.1 | 72.2 | 8.6 | 0 |
| | *Streptomyces albulus* NBRC14147 | ε-poly-L-lysine | BHXC0000000 | 72.2 | 9.6 | 0 |
| | *Streptomyces coelicolor* A3(2) | Actinorhodin | AL645882.2 | 72.1 | 8.7 | 0 |
| | *Streptomyces rimosus* ATCC 10970 | Oxy tetracycline | NZ_CP048261.1 | 72 | 9.4 | 0 |
| | *Streptomyces spectabilis* ATCC 27465 | Spectinomycin | NZ_CP023690.1 | 72 | 9.8 | 0 |
| | *Streptomyces ambofaciens* ATCC 23877 | Spiramycin | NZ_CP012382.1 | 72 | 8.3 | 1 |
| | *Streptomyces kanamyceticus* ATCC 12853 | Kanamycin | NZ_CP023699.1 | 71 | 10.1 | 7 |
| | *Streptomyces linconensis* LC-G | Lincomycin | NZ_CP0022744.1 | 71 | 9.5 | 1 |
| | *Streptomyces avermitilis* MA-4680T | Avermectin | NC_003155.5 | 70.7 | 9 | 0 |
| *Non-streptomyces* | *Saccharopolyspora erythraea* NRRL 23338 | Erythromycin | NC_009142.1 | 71.1 | 8.2 | 1 |
| | *Amycolaptasis orientalis* B-37 | Vancomycin | NZ_CP016174.1 | 69 | 9.5 | 0 |
| | *Saccharopolyspora spinosa* CCTCC M206084 | Spinosyn | NZ_CP061007.1 | 68 | 8.9 | 0 |

From these results, it was expected that by introducing a PacI recognition sequence in advance near the target gene and heterologously expressing PacI in the actinomycete host, site-specific DSB can be induced, and through subsequent homologous recombination repair, the desired region can be deleted without leaving drug resistance genes or sequences derived from heterologous organisms.

Here, Cas9 derived from Streptococcus pyogenes, which is conventionally used extensively for genome editing, tolerates mismatches in the protospacer-adjacent motif (PAM) at a certain level. Therefore, in editing actinomycete genomes with high GC content and many repetitive sequences, it involves risks such as frequent off-target editing and toxicity expression due to non-specific cleavage of the host genome. Additionally, since Cas9 has 1368 amino acid residues and a large molecular weight, the size of the editing plasmid inevitably becomes large, which also becomes one of the barriers for genome editing in actinomycetes which are microorganisms that are difficult to transform.

On the other hand, PacI is a homodimer enzyme composed of polypeptide chains with only 142 amino acid residues, which is the smallest among known nucleases, making it possible to significantly reduce the size of the editing plasmid. Accordingly, by performing genome editing with the method using PacI, it becomes possible to conduct rational molecular breeding without causing off-target effects, and this can be expected to greatly contribute to the development of natural product drug discovery in the future.

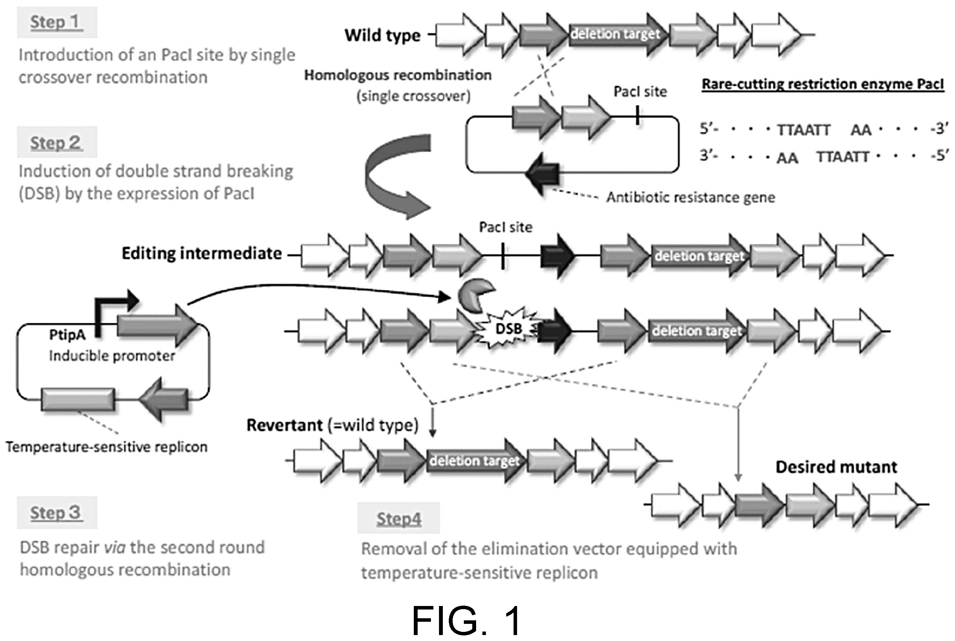

Therefore, the inventors proceeded with studies on genome editing using PacI and conceived of performing genome editing based on the method exemplified below (FIG. 1).

First, in the case of deleting a target nucleotide sequence by genome editing, sequences linking the flanking regions on the 5' side and 3' side of the target nucleotide sequence in the genome editing target region are amplified by PCR method to prepare homologous arms and insert into a Mobilization vector containing a PacI site, thereby constructing a vector for introducing a type II restriction endonuclease recognition sequence into the editing target region (hereinafter also referred to as Targeting vector). By introducing this vector into a host, an editing intermediate in which the full length of the vector containing the PacI site is inserted into the genome by single crossover homologous recombination is obtained. Subsequently, a PacI expression vector is additionally introduced, PacI expression is induced by inducer addition, and specific DSB is induced. Thereafter, an editing completion strain that has reverted by homologous recombination repair using the previously introduced homologous arms is selected. Finally, the PacI expression vector equipped with a temperature sensitive origin of replication is eliminated from the editing completion strain by high-temperature culture, and an editing completion strain containing no foreign genes is obtained. Further, the design also enables multiplex genome modification in a semi-one-step manner by additionally introducing the next Targeting vector while retaining the PacI expression vector.

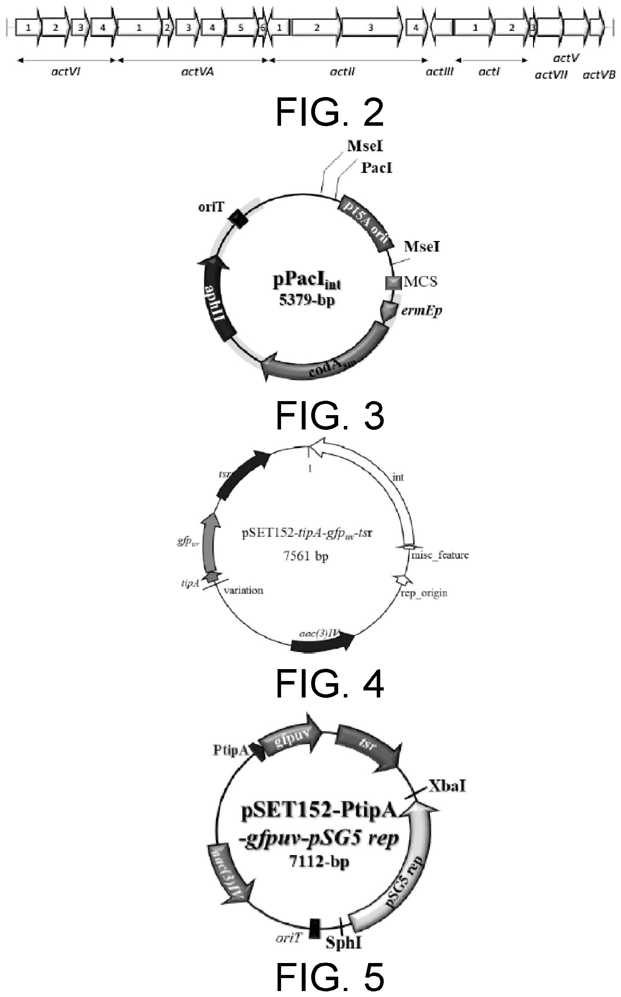

In validation experiments of the genome editing principle in actinomycetes, a method of visually determining wild-type strains and editing completion strains on plates by selecting biosynthesis genes of pigmented antibiotics (actinorhodin and undecylprodigiosin (RED)) as target genes is widely used. Therefore, the inventors used *Streptomyces coelicolor* A3(2) (strain name: Streptomyces violaceoruber NBRC15146), which is a high-production model actinomycete of actinorhodin, a blue antibiotic, selected the actinorhodin biosynthesis gene cluster (21.6 kbp) in the actinomycete as the target gene (FIG. 2), and conducted validation experiments of the above genome editing principle by performing scarless editing of the actinorhodin biosynthesis gene cluster.

### <Example 2 Construction of Genome Editing Vector>

### 1. Construction of PacI Recognition Sequence Introduction Vector (pPacIᵢₙₜ)

It is considered that in the genome editing process, editing intermediate strains may remain at a certain frequency, but by introducing a negative selection marker gene into the editing intermediate strain in advance together with the PacI recognition sequence, unedited intermediate strains can be eliminated and editing completion strains can be selectively obtained. Regarding the negative selection systems in actinomycete strains, for example, it is known that a codon-optimized escherichia coli-derived cytosine deaminase (codA) gene functions as a negative selection marker in the presence of 5-fluorocytosine (5-FC) in several actinomycete strains.

DNA was chemically synthesized in which a codA gene to be used as a negative selection marker was placed downstream of a constitutive expression promoter (PermE). This codA gene has been codon-optimized to enhance translation efficiency within actinomycete cells. By methods known to those skilled in the art, this synthetic gene (PermE-codAsm (SEQ ID NO: 1)) was amplified by PCR using primers shown in Table 2, and then inserted into the NheI-HindII site of pK18mob to construct pK18mob-codAsm.

### [Table 2]

**Table 2**

| Primer | Sequence (5'-3') |
|---|---|
| codA-hindF (SEQ ID NO: 2) | ggcatgcaagcttattcgatatcatgtcggaccggag |
| codA-nheR (SEQ ID NO: 3) | gcgtgaagctagctcagcgcttgtagtcgatcgc |

Next, the origin of replication p15A ori in the 848bp to 1393bp region of pACYC184 (Accession No.: X06403) was amplified by PCR using primers shown in Table 3. This amplification product is designed so that a PacI recognition sequence is provided to the upstream side.

### [Table 3]

**Table 3**

| Primer | Sequence (5'-3') |
|---|---|
| p15A ori-F (SEQ ID NO: 4) | atctcatgaccaaaatcccttaattaagtgcgtcgggtgatgctgcc |
| **pl5A ori-R** (SEQ ID NO: 5) | tgagtgagctaactcacattaatttaactgtgataaactaccgc |

pK18mob-codA-sm that had been previously digested and linearized with MseI was mixed with p15A ori amplified by PCR, and ligated by Gibson assembly method. This vector was designated as pPacIᵢₙₜ (SEQ ID NO: 6, FIG. 3) and used in subsequent validation experiments of the effectiveness of the present genome editing system.

### 2. Construction of PacI Expression Vector (pELIM)

### (1) Confirmation of Effectiveness of Thiostrepton-inducible Promoter based on gfp Gene Expression

To induce DSB introduction by expressing PacI in a time-specific manner within a wide range of actinomycete host cells, a broad-host-range inducible promoter is required. The effectiveness of thiostrepton-inducible PtipA has been known in several actinomycete strains. Therefore, based on the sequence of pIJ6021 plasmid (Accession No. AJ414669), a thiostrepton-inducible promoter (PtipA (SEQ ID NO: 7)) was constructed by oligonucleotide assembly using primers shown in Table 4.

### [Table 4]

**Table 4**

| Primer | Sequence (5'-3') |
|---|---|
| PtipA-EcoRV-1F (SEQ ID NO: 8) | tatgacatgattacgaattcgatatcagggctacgctggaccggatgggctgag |
| PtipA-2R (SEQ ID NO: 9) | cgttccgcgtgccacgccgtgagccgccgcgtgccgtcggctccctcagcccatccggtccagc |
| PtipA-3F (SEQ ID NO: 10) | acggcgtggcacgcggaacgtccgggcttgcacctcacgtcacgtgaggaggcagcgtggacgg |
| PtipA-NdeI-4R (SEQ ID NO: 11) | cccttgctcatatgctccgctcccttctctgacgccgtccacgctgcctcctcac |

Subsequently, using codon-optimized/chemically synthesized gfpᵤᵥ gene (SEQ ID NO: 12) as a template, amplification was performed by PCR using primers shown in Table 5.

### [Table 5]

**Table 5**

| Primer | Sequence (5'-3') |
|---|---|
| gfp-ndeF (SEQ ID NO: 13) | gcggagcatatgagcaagggcgaggagct |
| gfp-kpnR (SEQ ID NO: 14) | tggagatctagcggtaccggctcacttgtagagctcgtccatcc |

Using pLAE001 plasmid (SEQ ID NO: 15) constructed in Patent Document 1 as a template, thiostrepton resistance gene (tsr (SEQ ID NO: 16)) was amplified by PCR using primers shown in Table 6.

### [Table 6]

**Table 6**

| Primer | Sequence (5'-3') |
|---|---|
| tsr-kpnF (SEQ ID NO: 17) | gccggtaccgctagatctccaatatgcggggatcgaccgcg |
| tsr-xbaR (SEQ ID NO: 18) | agcttgggctgcaggtcgactctagattatcggttggccgcgagattc |

pSET152 plasmid (Accession No. AJ414670) that had been digested and linearized with Xbal was mixed with PtipA, gfpᵤᵥ, and tsr genes amplified by PCR, and ligated by Gibson assembly method to construct pSET152-PtipA-gfpᵤᵥ-tsr (SEQ ID NO: 19) capable of inducible expression of GFP under PtipA control (FIG. 4).

After completion of genome editing, the type II restriction endonuclease expression vector needs to be eliminated from cells. Here, it is known that modified plasmids carrying the origin of replication of temperature sensitive plasmid pSG5 (pSG5rep) are efficiently eliminated from various actinomycete host strains by culturing at high temperatures of 39°C or higher. Therefore, the φC31 integrase element of pSET152-PtipA-gfpᵤᵥ-tsr was replaced with pSG5rep. Based on the sequence of pSG5 (Accession No. NC_008792.1), pSG5rep (SEQ ID NO: 20) was chemically synthesized (commissioned to Eurofins Genomics K.K.). The pSG5rep fragment was excised by double restriction digestion using Xbal and SphI from pEX-A2J2 plasmid into which chemically synthesized pSG5rep had been cloned. This was ligated with pSET152-PtipA-gfpᵤᵥ-tsr from which the φC31 integrase element had been excised by the same restriction enzyme treatment, to construct pSET152-PtipA-gfpᵤᵥ-pSG5rep (hereinafter also described as pSET152-PtipA-gfp-tsr-pSG5rep (SEQ ID NO: 21)) (FIG. 5).

Next, the functionality of PtipA was evaluated based on the expression of GFPuv. The constructed pSET152-PtipA-gfp-tsr-pSG5rep was introduced into *Streptomyces albulus* NBRC14147 (National Institute of Technology and Evaluation (NITE) Biological Resource Center (NBRC)), Streptomyces lividans TK23 (ATCC), and *Streptomyces coelicolor* A3(2) (strain name: Streptomyces violaceoruber NBRC15146) (National Institute of Technology and Evaluation (NITE) Biological Resource Center (NBRC)) by conjugative transfer via E.coli ET12567 (ATCC). Each transformant was inoculated into TSBY liquid medium (TSB medium (Becton, Dickinson and Company) supplemented with 0.5% yeast extract) containing apramycin (Apr) (Sigma-Aldrich, 50 µg/ml) and cultured with shaking at 30°C until reaching stationary phase. After subculturing into fresh TSBY liquid medium containing Apr (50 µg/ml) and culturing with shaking at 30°C for 8 hours, various concentrations of thiostrepton (Sigma-Aldrich) were added to induce expression of GFPuv. After thiostrepton addition, the cultures were further shaken for 24 hours, and bacterial cells were collected by centrifugation.

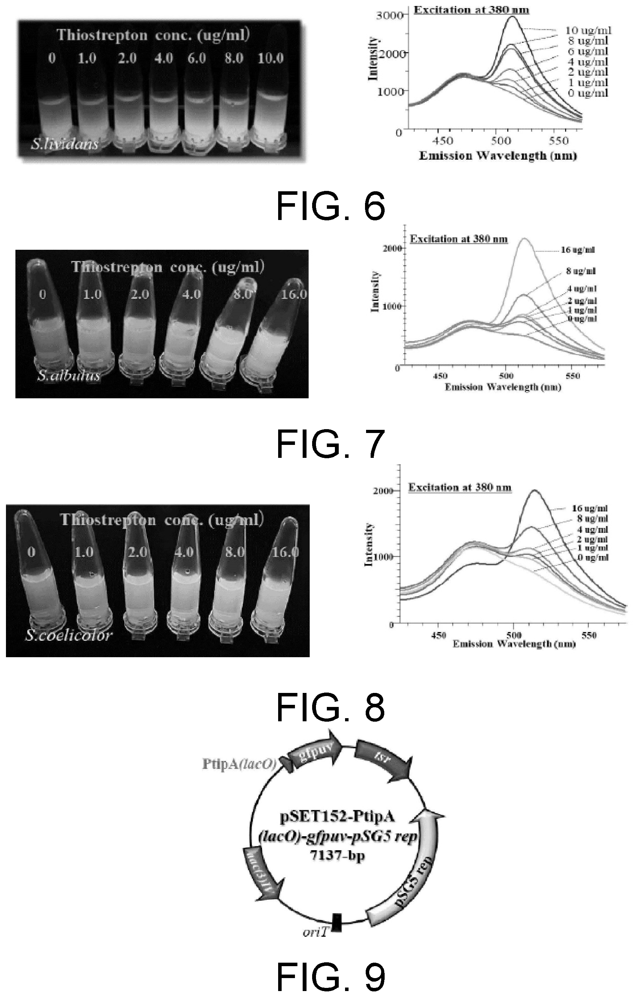

In response to measuring the fluorescence at excitation wavelength 380 nm of cell-free extract prepared by ultrasonic disruption, fluorescence around 510 nm derived from GFPuv was enhanced in a thiostrepton concentration-dependent manner in all strains (FIG. 6 to FIG. 8), confirming that PtipA can be used to control expression of downstream genes in a thiostrepton concentration-dependent manner within various actinomycete hosts.

### (2) Confirmation of Temperature Sensitivity based on pSG5rep

Next, temperature sensitive replication of pSET152-PtipA-gfp-tsr-pSG5rep within various actinomycetes was also verified. pSET152-PtipA-gfpᵤᵥ-pSG5rep was introduced into *Streptomyces albulus* NBRC14147, Streptomyces lividans TK23, and *Streptomyces coelicolor* A3(2) (strain name: Streptomyces violaceoruber NBRC15146) by conjugative transfer via E. coli ET12567, and transformants were selected based on apramycin resistance. Each transformant was cultured with shaking (30°C) for 48 hours in TSBY liquid medium containing apramycin. The culture solution that reached stationary phase was applied to agar medium containing no antibiotics and cultured at 39°C for 72 hours to induce plasmid loss.

Further, as a control experiment, the same culture solution was applied to agar medium containing apramycin and cultured at 39°C for 3 days. As a result, cell growth in the presence of apramycin was remarkably poor, and it was considered that plasmid loss was induced by high-temperature culture. Additionally, it was confirmed that the recombinant strains in which plasmid loss was induced on agar medium containing no antibiotics were apramycin-sensitive and did not retain the plasmid according to determination using PCR method.

From the above results, it was confirmed that pSET152-PtipA-gfp-tsr-pSG5rep functions as a temperature sensitive plasmid as designed.

### (3) Examination of pacI Expression Vector

Therefore, next, the pacI gene was cloned downstream of PtipA in the pSET152-PtipA-gfp-tsr-pSG5rep plasmid, and construction of a pacI expression vector was initiated. A synthetic pacI gene (SEQ ID NO: 22) codon-optimized for actinomycetes was obtained based on the sequence of pacI (Accession No. 3LDY_A) derived from Pseudomonas alcaligenes. Using this synthetic gene as a template, PCR reaction was performed using primers in Table 7 to obtain a pacI gene fragment with NdeI and BglII recognition sequences linked at both ends.

### [Table 7]

**Table 7**

| Primer | Sequence (5'-3') |
|---|---|
| pacI-ndeF (SEQ ID NO: 23) | agcggacgcatatgacgcagtgcccgcgctg |
| pacI-bglR (SEQ ID NO: 24) | tgcacatgaacctaggcgatcccccgcccgtg |

The gfpᵤᵥ gene was excised by double restriction digestion of pSET152-PtipA-gfp-tsr-pSG5rep with NdeI and BglII, and the pacI gene digested with NdeI and BglII was ligated using T4 DNA ligase. This ligation product was used to transform cloning E. coli host NEB10β (New England Biolabs), but the obtained transformants were very few. Additionally, in response to confirming the sequences of plasmids obtained from multiple transformants, unexpected nucleotide substitution and deletion were observed in the pacI gene region in all cases. This suggested the possibility that the pacI gene placed downstream of PtipA was expressed at basal level within the cloning escherichia coli host, resulting in cleavage of the host genome.

Here, it is known that lac repressor (lacI (SEQ ID NO: 25)) overexpressed within escherichia coli can control gene expression directly downstream of a promoter by binding to an operator sequence (lacO (SEQ ID NO: 26)) inserted between the -10 region of the promoter and the transcription start site. Therefore, controlling basal expression from PtipA by inserting lacO between the -10 region of PtipA and the transcription start site was examined.

Using the constructed pSET152-PtipA-gfp-tsr-pSG5rep as a template, PtipA with lacO sequence inserted (PtipA(lacO) (SEQ ID NO: 27)), gfpᵤᵥ gene, and tsr gene were amplified by PCR method. The primers used for amplification by this PCR method are shown in Table 8.

**[Table 8]**

| Table 8 Primers used in amplification of PtipA (PtipA(lacO)), gfpᵤᵥ gene, and tsr gene | | |
|---|---|---|
| Target region | Primer | Sequence (5'→3') |
| PtipA(lacO) | tipA-1F (SEQ ID NO: 28) | tatgacatgattacgaattcgatatcagggctacgctggaccggatgggctgag |
| | tipA-(lacO)-R (SEQ ID NO: 29) | attgttatccgctcacaattccacgctgcctcctcacgtgac |
| gfpᵤᵥ | gfpᵤᵥ₋(lacO)-F (SEQ ID NO: 30) | aattgtgagcggataacaattccggacggcgtcagagaagggag |
| | gfpᵤᵥ-MCS-R (SEQ ID NO: 31) | tggagatctagcggtaccggctcacttgtagagctcgtccatcc |
| tsr | tsr-MCS-F (SEQ ID NO: 32) | gccggtaccgctagatctccaatatgcggggatcgaccgcg |
| | tsr-XbaI-R (SEQ ID NO: 33) | agcttgggctgcaggtcgactctagattatcggttggccgcgagattc |

These DNA fragments were mixed with pSET152 that had been digested and linearized with EcoRV and Xbal, and ligated by Gibson assembly method to construct pSET152-PtipA(lacO)-gfpᵤᵥ-tsr. Furthermore, according to the method described above, pSET152-PtipA(lacO)-gfp-tsr-pSG5rep (SEQ ID NO: 34) was constructed by replacing the φC31 integrase element of the vector backbone with pSG5rep (FIG. 9).

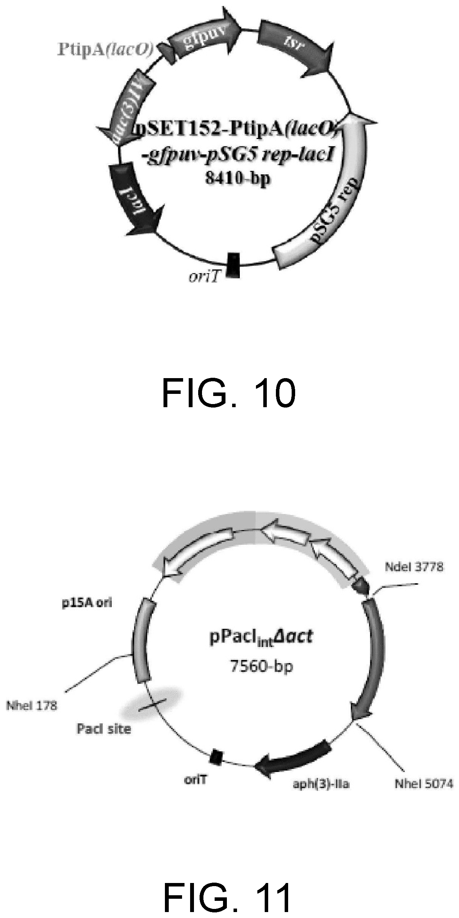

Next, the lacI gene was additionally introduced into pSET152-PtipA (lacO)-gfpᵤᵥ-pSG5 rep. Using primers in Table 9, the lacI gene (including promoter region (SEQ ID NO: 35)) was amplified from pET21b(+) (Merck Millipore) by PCR method and inserted into the NheI site of pSET152-PtipA(lacO)-gfpᵤᵥ-pSG5rep to construct pSET152-PtipA(lacO)-gfpᵤᵥ-pSG5rep-lacI (FIG. 10).

### [Table 9]

**Table 9**

| Primer | Sequence (5'-3') |
|---|---|
| lacI-Nhel-F (SEQ ID NO: 36) | gactctagctagcctactgggctgcttcctaatgc |
| lacI-Nhel-R (SEQ ID NO: 37) | ctggccagctagccgagatcccggtgcctaatg |

Next, the pacI gene was cloned into pSET152-PtipA-gfpᵤᵥ-pSG5rep-lacI equipped with an expression control system using lac operator and lac repressor. Using the chemically synthesized pacI gene as a template and amplifying by PCR method using primers in Table 7, a pacI gene with NdeI and BglII sites ligated at both ends was obtained. The pacI gene and pSET152-PtipA(lacO)-gfpᵤᵥ-pSG5rep-lacI restriction digested with NdeI and BglII were ligated using T4-DNA ligase. A portion of this reaction solution was used to transform to E.coli NEB10β, and then transformants were selected based on apramycin resistance. At this time, basal expression of PacI was controlled by adding 5% (w/v) glucose to the LB liquid medium and LB agar medium used for cloning. Construction of pSET152-PtipA(lacO)-pacI-pSG5rep-lacI (SEQ ID NO: 38) was confirmed by restriction mapping with EcoRV and NheI double digestion and DNA sequencing of plasmids extracted and purified from transformants, and this was used as a pacI expression vector (pELIM) in subsequent validation experiments of the principle.

### <Example 3 Scarless Deletion of Actinorhodin Biosynthesis Gene Cluster using PacI Genome Editing System (Validation Experiment of the Principle)>

### (1) Introduction of PacI Recognition Sequence into Actinorhodin Biosynthesis Gene Cluster

Using the genome extracted from Streptomyces violaceoruber NBRC15146 (strain name: *Streptomyces coelicolor* A3(2)) as a template, the flanking regions of approximately 1 kbp each on the 5' side and 3' side of the actinorhodin biosynthesis gene cluster (SEQ ID NO: 39) (peripheral regions of sco5071 and sco5092 genes (SEQ ID NO: 40 and SEQ ID NO: 41, respectively)) were amplified by PCR method using primers shown in Table 10.

### [Table 10]

**Table 10**

| Primer | Sequence (5'-3') |
|---|---|
| sco5071-5092del-left-F (SEQ ID NO: 42) | agctatgaccatgattacgaattcatccactccagtacgcgtacg |
| sco5071-5092del-left-R (SEQ ID NO: 43) | cgcgagctgatccattgacagcggtgatccggtg |
| sco5071-5092del-right-F (SEQ ID NO: 44) | ccgctgtcaatggatcagctcgcggaggatgtcg |
| sco5092del-rightR (SEQ ID NO: 45) | tcgaataagcttgcatgcctgcaggaacgtccgcctggtcgagac |

pPacIᵢₙₜ that had been previously digested and linearized with EcoRI and PstI was mixed with the two 1 kbp fragments amplified by PCR method and ligated by Gibson assembly method. A portion of this reaction solution was used to transform E. coli NEB10β competent cells, and clones showing kanamycin (FUJIFILM Wako Pure Chemical) resistance were selected. Construction of the actinorhodin biosynthesis gene cluster-specific targeting vector (pPacIᵢₙₜΔact (SEQ ID NO: 46)) was confirmed by performing restriction mapping with NdeI and NheI on plasmids extracted and purified by plasmid extraction using methods known to those skilled in the art (FIG. 11).

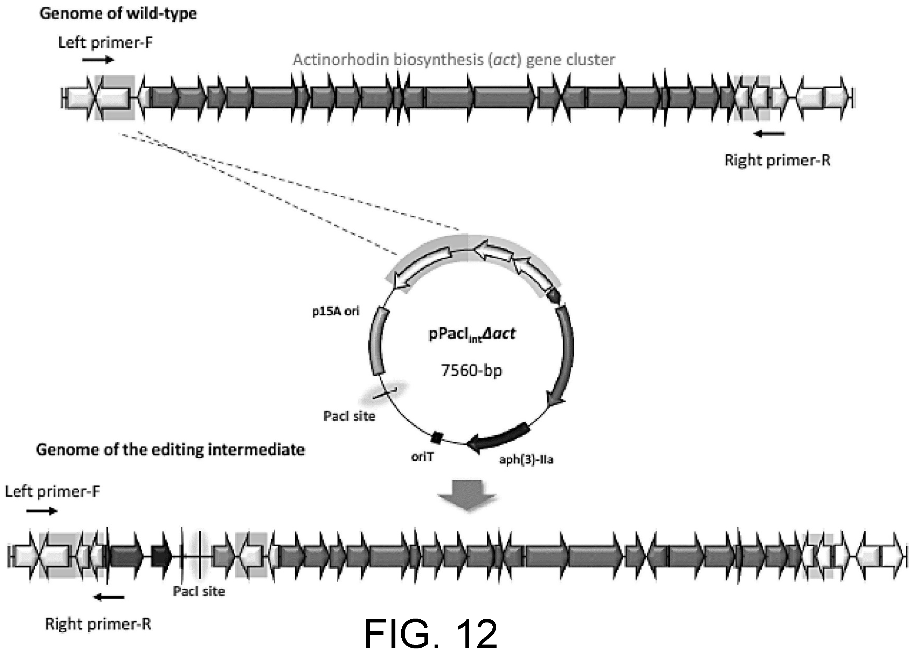

pPacIᵢₙₜΔact was introduced into Streptomyces violaceoruber NBRC15146 by conjugative transfer via E. coli S17-1 strain (ATCC). Using genomic DNA extracted from editing intermediate candidate strains showing neomycin (FUJIFILM Wako Pure Chemical) resistance as a template, verification by PCR method using primers shown in Table 11 was performed to confirm the construction of editing intermediate with PacI recognition sequence inserted into the genome as shown in the lower part of FIG. 12.

### [Table 11]

**Table 11**

| Primer | Sequence (5'-3') |
|---|---|
| sco5071-5092del-left-F (SEQ ID NO: 47) | agctatgaccatgattacgaattcatccactccagtacgcgtacg |
| sco5092del-rightR (SEQ ID NO: 48) | tcgaataagcttgcatgcctgcaggaacgtccgcctggtcgagac |

### (2) Scarless Deletion of Actinorhodin Biosynthesis Gene Cluster by PacI Expression

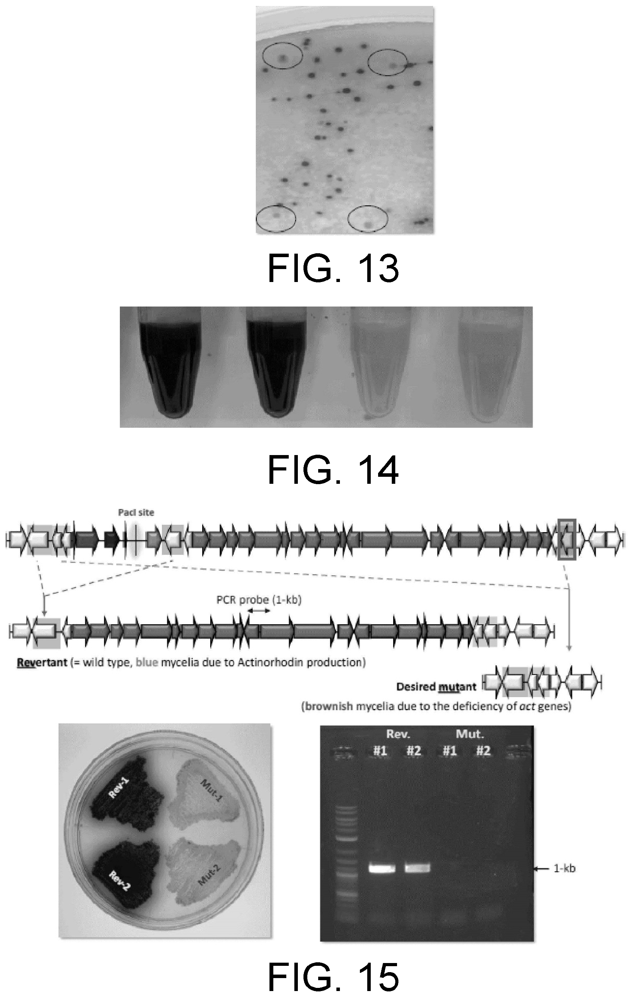

The constructed PacI expression plasmid pELIM was introduced into the editing intermediate strain by conjugative transfer via E. coli S17-1. Expression of PacI from pELIM is designed to be induced by thiostrepton addition. However, in response to selecting intermediate strains into which pELIM had been introduced using apramycin resistance as an indicator, it was confirmed that blue pigment production-deficient strains occurred without expression induction by thiostrepton addition (FIG. 13; colonies circled are blue pigment production-deficient strains).

This result suggested the possibility that immediately after introducing the vector, PacI was expressed at basal level without induction of expression by thiostrepton addition, and as a result of DSB being introduced into the genome, homologous recombination repair was induced and editing completion strains were generated. Therefore, two clones each of blue-colored colonies and brown colonies were picked and cultured in TSB (containing 10% sucrose) liquid medium for 3 days to confirm whether pigment was produced or not. As a result, the blue-colored clones showed vigorous actinorhodin production, while the brown clones were non-productive of actinorhodin, which also strongly suggested the possibility that editing completion strains with deleted actinorhodin biosynthesis gene cluster were generated simply by introducing pELIM (FIG. 14; the left two samples are actinorhodin-producing strains, and the right two samples are actinorhodin non-producing strains).

Therefore, genomic DNA was extracted from these bacterial cells, and verification by PCR method (genotype analysis) was performed using primers (Table 12) designed to amplify the central region of actinorhodin biosynthesis gene cluster (SEQ ID NO: 49).

### [Table 12]

**Table 12**

| Primer | Sequence (5'-3') |
|---|---|
| act-del-CK F (SEQ ID NO: 50) | cggtccgtcttcctgatcaatctg |
| act-del-CK R (SEQ ID NO: 51) | agcagtgtgcgtgtcatcagaag |

Further, pigment production (phenotype analysis) in R5 medium, which was a secondary metabolite production medium, was confirmed again. As a result of genotype analysis, amplification by PCR method was confirmed only in blue-colored colonies, which completely matched the results of phenotype analysis in R5 medium (FIG. 15).

From the above results, it was confirmed that the blue-colored clones, which accounted for approximately 92% of all transformants, were wild-type revertant strains, and the remaining 8% of brown clones were editing completion strains. In addition, since all blue and brown clones were neomycin-sensitive, it was also confirmed that no editing intermediates remained after pELIM introduction.

Next, to confirm whether the target region had been accurately deleted, sequence analysis was performed in the region surrounding the editing region. For this sequence analysis, primers shown in Table 13 were used.

### [Table 13]

**Table 13**

| Primer | Sequence (5'-3') |
|---|---|
| act-del-seq F1 (SEQ ID NO: 52) | tgggtacctgtgctgcttttcg |
| act-del-seq R1 (SEQ ID NO: 53) | agctcgtcgggaagcagcttc |
| act-del-seq F2 (SEQ ID NO: 54) | gtccgtgcacgtcgaggagaag |
| act-del-seq R2 (SEQ ID NO: 55) | cactacctccacacgcccttc |

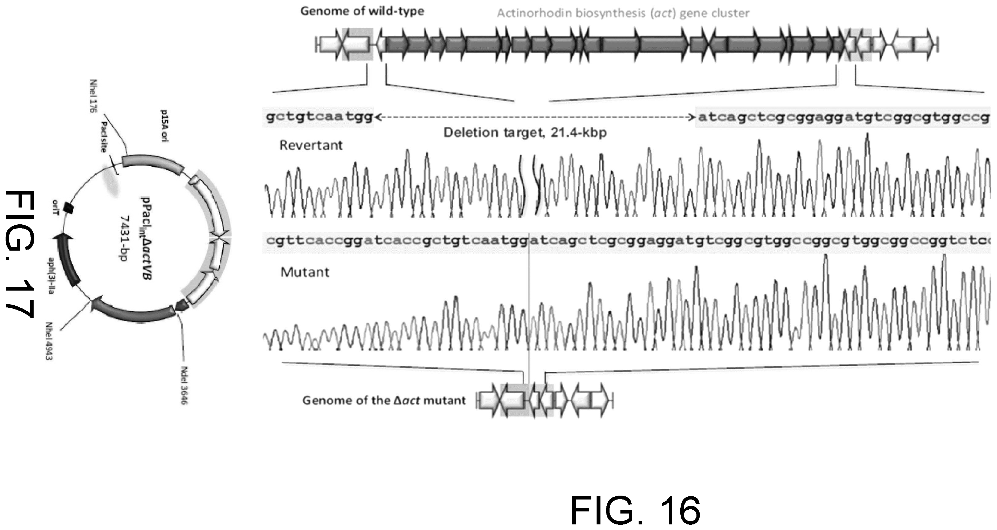

For wild-type revertant strains, the 5' side flanking region and 3' side flanking region of the actinorhodin biosynthesis gene cluster were analyzed, and for editing completion strains, the junction region between the 5' side flanking region and 3' side flanking region was analyzed. As a result, it was confirmed that blue-colored clones had completely reverted to the wild-type sequence, while brown clones had accurately deleted the target region as designed (FIG. 16). Furthermore, by culturing the editing completion strain at 40°C for 5 days, apramycin-sensitive clones from which pELIM had been eliminated could also be obtained.

Through the above validation experiments of the principle, it was demonstrated that the present method using type II restriction enzyme PacI can specifically introduce DSB into actinomycete genomes, and accurate genome editing can be achieved without leaving foreign genes.

Next, to confirm the influence of deletion length on genome editing efficiency, deletion of actVB (560 bp) located at the terminal of the biosynthesis gene cluster was performed. Using the genome extracted from Streptomyces violaceoruber NBRC15146 as a template, the flanking regions of approximately 1 kbp each on the 5' side and 3' side of actVB (SEQ ID NO: 56) (SEQ ID NO: 57 and SEQ ID NO: 58, respectively) were amplified by PCR method using primers shown in Table 14.

### [Table 14]

**Table 14**

| Primer | Sequence (5'-3') |
|---|---|
| sco5092del-leftF (SEQ ID NO: 59) | agctatgaccatgattacgaattcgcggacgcccgtacccatcgt |
| sco5092del-lefrR (SEQ ID NO: 60) | cgagctgatcgctgccatcttcgaactccctag |
| sco5092del-rightF (SEQ ID NO: 61) | agatggcagcgatcagctcgcggaggatgct |
| sco5092del-rightR (SEQ ID NO: 62) | tcgaataagcttgcatgcctgcaggaacgtccgcctggtcgagac |

The obtained two types of PCR fragments were mixed with a Mobilization vector (pPacIᵢₙₜ) that had been previously digested and linearized with EcoRI and PstI, and ligated by Gibson assembly method. A portion of this reaction solution was used to transform E. coli NEB10β competent cells, and transformants were selected based on kanamycin resistance. Construction of the actVB gene cluster-specific Targeting vector (pPacIᵢₙₜΔactVB (SEQ ID NO: 63)) was confirmed by performing restriction mapping with NdeI and NheI using plasmids extracted and purified from transformants (FIG. 17).

This pPacIᵢₙₜΔactVB was introduced into Streptomyces violaceoruber NBRC15146 by conjugative transfer via E. coli S17-1 strain. Using genomic DNA extracted from editing intermediate candidate strains showing neomycin resistance as a template, verification was performed by PCR method using primers shown in Table 15, confirming the construction of editing intermediate Streptomyces violaceoruber NBRC15146/pPacIᵢₙₜΔactVB in which the PacI recognition sequence had been inserted into the genome.

### [Table 15]

**Table 15**

| Primer | Sequence (5'-3') |
|---|---|
| sco5092del-seqF (SEQ ID NO: 64) | tccgttcgccctcttcggttc |
| sco5092del-seqR (SEQ ID NO: 65) | ctcaaccacctgacccacgtc |

Subsequently, to induce genome editing by PacI expression, pELIM was introduced into Streptomyces violaceoruber NBRC15146/pPacIᵢₙₜΔactVB by conjugative transfer via E. coli S17-1 strain. Since deletion of only the actVB gene located at the terminal of the biosynthesis gene cluster does not result in loss of pigment production ability, success or failure of genome editing cannot be confirmed based on phenotype. Therefore, 20 clones of pELIM-introduced strains showing apramycin resistance were randomly selected, and verification was performed by PCR method using primers shown in Table 16.

### [Table 16]

**Table 16**

| Primer | Sequence (5'-3') |
|---|---|
| actVB-del-CK-F (SEQ ID NO: 66) | tggaagaggtcctggggaaag |
| actVB-del-CK-R (SEQ ID NO: 67) | aaacgactgaaggagttccgatgag |

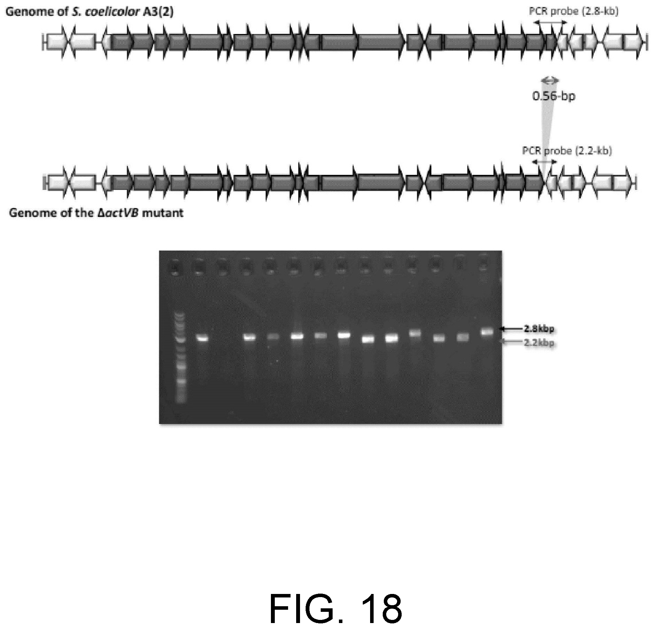

As a result, it was confirmed that 12 clones were wild-type revertant strains and 8 clones were actVB gene deletion strains (FIG. 18). Further, similar to the case of full-length deletion of the actinorhodin biosynthesis gene cluster, it was confirmed that no editing intermediates remained after pELIM introduction.

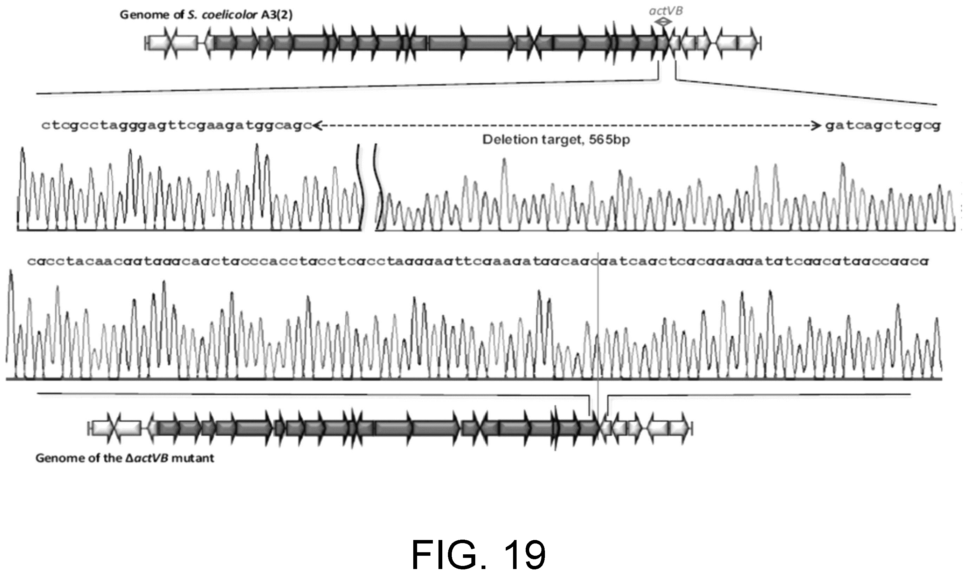

Further, as shown in FIG. 19, sequence analysis around the editing region confirmed that all blue-colored clones had completely reverted to the original wild-type sequences rather than being editing intermediates, and that brown clones had accurately deleted the actVB gene region without even a single nucleotide deviation.

In the deletion of actVB gene (560 bp) on the 3' terminal side of the actinorhodin biosynthesis gene cluster, 8 out of 20 clones were editing completion strains, whereas in the full-length deletion of the actinorhodin biosynthesis gene cluster where visual determination by pigment was possible, only 4 out of all 48 clones showed brown color indicating editing completion. From the above results, it was confirmed that there is a tendency for the frequency of generating wild-type revertant strains to increase in long-distance gene deletion.

The present invention successfully established a new genome editing technique platform that avoids off-target effects, which has been the greatest challenge in genome editing of actinomycetes. Since the present invention uses a combination of promoters and temperature sensitive origin of replications that function in a wide range of actinomycete species, the present invention can be applied not only to the actinomycete strains exemplified in the examples, but also to the breeding of a broad range of industrial actinomycete strains. Therefore, the further improvement of this method is expected to pave the way for molecular breeding of a broad range of industrial actinomycete strains.

### <Example 4 Construction of Single Vector-Type PacI Genome Editing System and Scarless Deletion of Actinorhodin Biosynthesis Gene Cluster Using the Same>

Although the effectiveness of two-stage genome editing using two types of plasmids, pPacIᵢₙₜ plasmid and pELIM plasmid, was demonstrated, it requires approximately 40 days for two rounds of plasmid introduction into actinomycetes that are difficult to transform, followed by selection of editing completion strains, and further elimination of pELIM. Therefore, an attempt was made to accelerate the entire process by performing one-stage genome editing in which only the elements essential for editing were integrated into a single vector.

It was decided to construct an actinorhodin biosynthesis gene cluster-specific editing vector that enables insertion of PacI recognition sequence into the genome, induction of double-strand breaking by PacI expression, and editing of the desired region by homologous recombination repair with a single vector, by adding the actinorhodin biosynthesis gene cluster-targeting sequences of pPacIᵢₙₜΔact (SEQ ID NO: 46) (flanking regions of sco5071 and sco5092 genes (SEQ ID NO: 40 and SEQ ID NO: 41, respectively)) and PacI recognition sequence to pELIM.

The flanking regions of approximately 1 kbp each on the 5' side and 3' side of the actinorhodin biosynthesis gene cluster (SEQ ID NO: 39) (flanking regions of sco5071 and sco5092 genes (SEQ ID NO: 74 and SEQ ID NO: 75, respectively)) were amplified by PCR method using primers shown in Table 17.

### [Table 17]

**Table 17**

| Primer | Sequence (5'-3') |
|---|---|
| sco5071-5092del-left-F2 (SEQ ID NO: 76) | cgataatctagattaattaaatccactccagtacgcgtacg |
| sco5071-5092del-left-R (SEQ ID NO: 43) | cgcgagctgatccattgacagcggtgatccggtg |
| sco5071-5092del-right-F (SEQ ID NO: 44) | ccgctgtcaatggatcagctcgcggaggatgtcg |
| sco5071-5092del-right-R2 (SEQ ID NO: 77) | ctgcaggcatgcgaacgtccgcctggtcgagac |

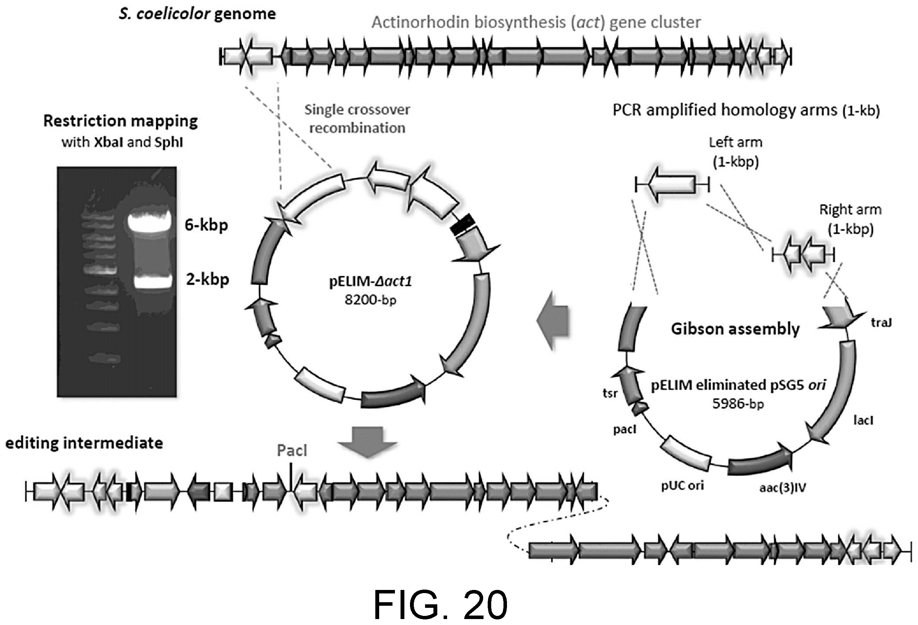

pELIM that had been previously digested and linearized with Xbal and SphI was mixed with two 1 kbp fragments amplified by PCR method, and ligated by Gibson assembly method. A portion of this reaction solution was used to transform E. coli NEB10β competent cells, and clones showing apramycin resistance were selected. Construction of a single actinorhodin biosynthesis gene cluster-specific editing vector (pELIM-Δact1 (SEQ ID NO: 78)) was confirmed by performing restriction mapping with XbaI and SphI on plasmids extracted and purified by plasmid extraction using methods known to those skilled in the art (FIG. 20).

pELIM-Δact1 was introduced into Streptomyces violaceoruber NBRC15146 by conjugative transfer via E. coli S17-1 strain (ATCC). Single crossover homologous recombinant strains showing apramycin resistance were selected as editing intermediates with the full-length pELIM-Δact1 inserted into the genome.

Selected colonies were scratched several times with the tip of a sterile toothpick, and then transferred by streaking onto agar medium containing 2 mM IPTG. After culturing this at 30°C for 3 days, it was confirmed that blue pigment production-deficient strains were generated as expected.

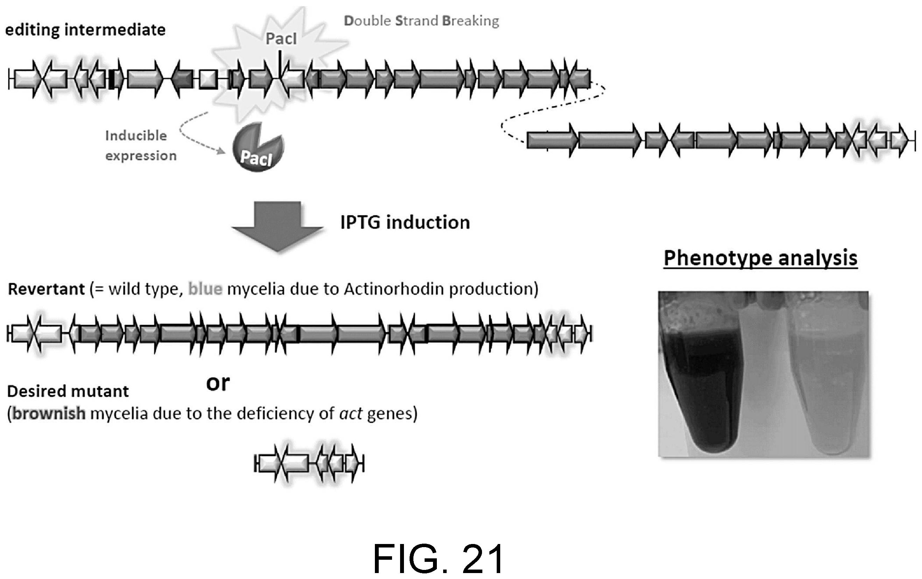

Two clones each of blue-colored colonies and brown colonies were picked and cultured in TSB (containing 10% sucrose) liquid medium for 3 days to confirm whether pigment was produced or not. As a result, the blue-colored clones showed vigorous actinorhodin production, while the brown clones were non-productive of actinorhodin, which also strongly suggested the possibility that deletion of the actinorhodin biosynthesis gene cluster was induced by a single editing vector (FIG. 21).

Therefore, next, genomic DNA was extracted from these bacterial cells, and verification by PCR method (genotype analysis) was performed using primers designed to amplify the central region of the actinorhodin biosynthesis gene cluster (SEQ ID NO: 49) (primers described in Table 12 above), and the region was not detected. Further, sequence analysis of the editing flanking region was performed using primers shown in Table 13 above, and as a result, it was confirmed that the target region could be accurately deleted in the same manner as in the two-stage editing.

In the one-stage genome editing using this single vector, it was possible to perform all operations for editing with a single plasmid introduction, and furthermore, since the introduced editing plasmid did not remain in the cells after editing, plasmid curing operations were also unnecessary. In the two-stage genome editing using two types of plasmids, approximately 40 days were required from construction of the editing vector specific to the editing target region to completion of editing, but one-stage genome editing completed the editing in approximately 20 days.

In the one-stage genome editing, the editing efficiency was equivalent to that of two-stage genome editing. Although this example discloses specific scarless deletion (knockout) of actinomycete genome using the type II restriction enzyme PacI, it is also possible to insert any sequence into a desired region (knock-in) by pre-inserting any sequence between the homologous arms provided to the editing vector.

## Claims

1. A method for deleting, substituting or inserting a target nucleotide sequence in a genome editing target region in an organism or cell to be subjected to genome editing, the method comprising:
(1) a step of introducing a type II restriction endonuclease recognition sequence into the genome editing target region; and
(2) a step of expressing a type II restriction endonuclease that recognizes the introduced type II restriction endonuclease recognition sequence in the organism or cell to induce double-strand breaking (DSB) in a genome,
wherein after performing the steps (1) and (2), a target region is edited through homologous recombination repair with a genome editing gene sequence in the organism or cell, and
a GC content in the genome of the organism or cell is 65% or more.

2. The method according to claim 1, wherein the type II restriction endonuclease recognizes a nucleotide sequence composed only of A and T.

3. The method according to claim 1, wherein the type II restriction endonuclease is PacI.

4. The method according to claim 1, wherein the organism is a eukaryote or a prokaryote that does not have the type II restriction endonuclease recognition sequence in the genome.

5. The method according to claim 4, wherein the organism is an actinomycete.

6. The method according to claim 5, wherein the actinomycete is an actinomycete that does not have a recognition sequence for PacI in the genome.

7. The method according to claim 5, wherein the actinomycete is an actinomycete selected from the group consisting of genus Streptomyces, genus Saccharopolyspora, and genus Amycolaptosis.

8. The method according to claim 5, wherein the actinomycete is an actinomycete selected from the group consisting of *Streptomyces fradiae*, *Streptomyces venezuelae, Streptomyces griseus, Streptomyces albulus, Streptomyces coelicolor, Streptomyces rimosus*, *Streptomyces spectabilis*, *Streptomyces avermitilis*, *Amycolatopsis orientalis*, and *Saccharopolyspora spinosa.*

9. The method according to any one of claims 1 to 8, wherein introduction in the step (1) is performed using an introduction vector for the type II restriction endonuclease recognition sequence, and
the introduction vector is a vector comprising (i) the genome editing gene sequence, (ii) the type II restriction endonuclease recognition sequence, (iii) an origin of replication sequence that operates in a host organism used for cloning the introduction vector and does not operate in the organism or cell to be subjected to genome editing, and (iv) a drug resistance gene sequence.

10. The method according to claim 9, wherein the origin of replication sequence is a p15A ori sequence.

11. The method according to claim 9, wherein the introduction vector further comprises a constitutive expression promoter sequence in the organism or cell to be subjected to genome editing and a negative selection gene sequence operably arranged downstream thereof, and
optionally, the negative selection gene sequence is codon-optimized.

12. The method according to claim 11, wherein the constitutive expression promoter sequence is an ermE promoter (PermE) sequence, and/or
the negative selection gene sequence is a codA gene sequence.

13. The method according to claim 11, further comprising:
(3) a step of selecting a cell into which the type II restriction endonuclease recognition sequence has been introduced using the negative selection gene.

14. The method according to any one of claims 1 to 8, wherein the step (2) is performed using an expression vector for the type II restriction endonuclease that recognizes the introduced type II restriction endonuclease recognition sequence, and
the expression vector is a vector comprising a promoter sequence that operates in the organism or cell to be subjected to genome editing and a type II restriction endonuclease sequence operably arranged downstream thereof, and
comprising an origin of replication sequence that operates in a host organism used for cloning the expression vector and does not operate in the organism or cell to be subjected to genome editing, a drug resistance gene sequence, and a sequence of temperature sensitive origin of replication that operates in the organism or cell to be subjected to genome editing.

15. The method according to claim 14, wherein the promoter sequence is an inducible expression promoter sequence,
the origin of replication sequence that operates in the host organism used for cloning the expression vector and does not operate in the organism or cell to be subjected to genome editing is a pUC ori sequence, and/or
the sequence of the temperature sensitive origin of replication that operates in the organism or cell to be subjected to genome editing is a pSG5rep sequence.

16. The method according to claim 15, wherein the inducible expression promoter sequence is a tipA promoter (PtipA) sequence.

17. The method according to claim 14, wherein the expression vector comprises an operator sequence and a repressor sequence,
the operator sequence is inserted between the promoter sequence and a transcription start site, and
optionally, the type II restriction endonuclease sequence is codon-optimized.

18. The method according to claim 17, wherein the operator sequence is a lacO sequence, and/or
the repressor sequence is a lacI sequence.

19. The method according to claim 14, further comprising:
(4) a step of eliminating the expression vector from the organism or cell based on temperature sensitivity of the temperature sensitive origin of replication.

20. The method according to any one of claims 1 to 8, being a method for performing introduction of the type II restriction endonuclease recognition sequence in the step (1) and expression of the type II restriction endonuclease that recognizes the type II restriction endonuclease recognition sequence in the step (2) with a single vector,
wherein the single vector is a vector comprising (i) the genome editing gene sequence, (ii) the type II restriction endonuclease recognition sequence, (iii) a promoter sequence that operates in the organism or cell to be subjected to genome editing and a type II restriction endonuclease sequence operably arranged downstream thereof, (iv) an origin of replication sequence that operates in a host organism used for cloning the single vector and does not operate in the organism or cell to be subjected to genome editing, and (v) a drug resistance gene sequence.

21. The method according to claim 20, wherein the promoter sequence is an inducible expression promoter sequence, and/or
the origin of replication sequence that operates in the host organism used for cloning the single vector and does not operate in the organism or cell to be subjected to genome editing is a pUC ori sequence.

22. The method according to claim 21, wherein the inducible expression promoter sequence is a tipA promoter (PtipA) sequence.

23. The method according to claim 20, wherein the single vector comprises an operator sequence and a repressor sequence,
the operator sequence is inserted between the promoter sequence and a transcription start site, and
optionally, the type II restriction endonuclease sequence is codon-optimized.

24. The method according to claim 23, wherein the operator sequence is a lacO sequence, and/or
the repressor sequence is a lacI sequence.

25. An introduction vector for type II restriction endonuclease recognition sequence, comprising (i) a genome editing gene sequence or a region into which the genome editing gene sequence is insertable, (ii) a type II restriction endonuclease recognition sequence, (iii) an origin of replication sequence that operates in a host organism used for cloning the introduction vector and does not operate in an organism or cell to be subjected to genome editing, and (iv) a drug resistance gene sequence.

26. The introduction vector according to claim 25, wherein the origin of replication sequence is a p15A ori sequence.

27. The introduction vector according to claim 25, further comprising a constitutive expression promoter sequence in the organism or cell to be subjected to genome editing and a negative selection gene sequence operably arranged downstream thereof, and
optionally, the negative selection gene sequence is codon-optimized.

28. The introduction vector according to claim 27, wherein the constitutive expression promoter sequence is an ermE promoter (PermE) sequence, and/or
the negative selection gene sequence is a codA gene sequence.

29. An expression vector for type II restriction endonuclease, comprising a promoter sequence that operates in an organism or cell to be subjected to genome editing and a type II restriction endonuclease sequence operably arranged downstream thereof, and
comprising an origin of replication sequence that operates in a host organism used for cloning the expression vector and does not operate in the organism or cell to be subjected to genome editing, a drug resistance gene sequence, and a temperature sensitive origin of replication sequence that operates in the organism or cell to be subjected to genome editing.

30. The expression vector according to claim 29, wherein the promoter sequence is a tipA promoter (PtipA) sequence,
the origin of replication sequence that operates in the host organism used for cloning the expression vector and does not operate in the organism or cell to be subjected to genome editing is a pUC ori sequence, and/or
the temperature sensitive origin of replication sequence that operates in the organism or cell to be subjected to genome editing is a pSG5rep sequence.

31. The expression vector according to claim 29, wherein the expression vector comprises an operator sequence and a repressor sequence,
the operator sequence is inserted between the promoter sequence and a transcription start site, and
optionally, the type II restriction endonuclease sequence is codon-optimized.

32. The expression vector according to claim 31, wherein the operator sequence is a lacO sequence, and/or
the repressor sequence is a lacI sequence.

33. A single vector for introducing a type II restriction endonuclease recognition sequence and for expressing a type II restriction endonuclease, comprising (i) a genome editing gene sequence or a region into which the genome editing gene sequence is insertable, (ii) a type II restriction endonuclease recognition sequence, (iii) a promoter sequence that operates in an organism or cell to be subjected to genome editing and a type II restriction endonuclease sequence operably arranged downstream thereof, (iv) an origin of replication sequence that operates in a host organism used for vector cloning and does not operate in the organism or cell to be subjected to genome editing, and (v) a drug resistance gene sequence.

34. The single vector according to claim 33, wherein the promoter sequence is a tipA promoter (PtipA) sequence, and/or
the origin of replication sequence that operates in the host organism used for cloning the single vector and does not operate in the organism or cell to be subjected to genome editing is a pUC ori sequence.

35. The single vector according to claim 33, wherein the single vector comprises an operator sequence and a repressor sequence,
the operator sequence is inserted between the promoter sequence and a transcription start site, and
optionally, the type II restriction endonuclease sequence is codon-optimized.

36. The single vector according to claim 35, wherein the operator sequence is a lacO sequence, and/or
the repressor sequence is a lacI sequence.

37. A kit for deleting, substituting or inserting a target nucleotide sequence in a genome editing target region in an organism or cell to be subjected to genome editing, the kit comprising:
(i) the introduction vector according to any one of claims 25 to 28, and the expression vector according to any one of claims 29 to 32, or
(ii) the single vector according to any one of claims 33 to 36.
